(19) Europäisches Patentamt
European Patent Office
Office européen des brevets

(11) EP 0 907 104 B1

(12) **EUROPEAN PATENT SPECIFICATION**

(45) Date of publication and mention of the grant of the patent:
**14.04.2004 Bulletin 2004/16**

(51) Int Cl.7: **G03C 7/42**, G03C 7/407, G03C 5/305, G03C 5/42, C07C 229/76

(21) Application number: **98118308.0**

(22) Date of filing: **25.09.1998**

# (54) Photographic processing composition and processing method

Photographische Verarbeitungszusammensetzung und photographisches Verarbeitungsverfahren

Composition et méthode de traitement photographique

(84) Designated Contracting States:
**AT BE CH CY DE DK ES FI FR GB GR IE IT LI LU MC NL PT SE**

(30) Priority: **26.09.1997 JP 26183697**

(43) Date of publication of application:
**07.04.1999 Bulletin 1999/14**

(73) Proprietor: **Fuji Photo Film Co., Ltd.**
**Kanagawa-ken (JP)**

(72) Inventors:
- **Inaba, Tadashi**
  **Minami-Ashigara-shi, Kanagawa-ken (JP)**
- **Kojima, Tetsuro**
  **Minami-Ashigara-shi, Kanagawa-ken (JP)**
- **Miyazaki, Hideo**
  **Minami-Ashigara-shi, Kanagawa-ken (JP)**

(74) Representative: **HOFFMANN - EITLE**
**Patent- und Rechtsanwälte**
**Arabellastrasse 4**
**81925 München (DE)**

(56) References cited:
**EP-A- 0 584 665**
**EP-A- 0 762 203**
**JP-A- 62 143 048**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### FIELD OF THE INVENTION

The present invention relates to a processing composition for a silver halide photographic light-sensitive material. Particularly, the present invention relates to a bleach-processing composition and a method of processing a silver halide color photographic light-sensitive material (hereinafter, also referred to as a light-sensitive material), that is excellent in desilvering, photographic properties, and image storage characteristics after the processing.

### BACKGROUND OF THE INVENTION

Light-sensitive materials are processed with a processing solution having a bleaching capacity and the like after they are exposed to light and subjected to color-development. As bleaching agents contained in the processing solution having a bleaching capacity, ferric complex salts are widely known, and particularly out of them, the ferric complex salt of ethylenediaminetetraacetic acid (EDTA) has long been used, and the ferric complex salt of 1,3-propanediaminetetraacetic acid (1,3-PDTA), having a more powerful bleaching capacity, has become used widely in recent years. Although, compared to the ferric complex salt of EDTA, the ferric complex salt of 1,3-PDTA is excellent in rapid processibility, bleach fogging is liable to occur because of its strong oxidation power, and the image storage characteristics after the processing is easily deteriorated (resulting in an increase in magenta stain). Further, since the oxidation-reduction potential is high, a system containing thiosulfuric acid has some problems because, for example, decomposition of thiosulfuric acid is facilitated with time, to deposit sulfur. Further, in view of the growing awareness of preservation of the earth's environment in recent years, in the photographic industry, wherein the development of processing agents low in environmental pollution load is required, the development of bleaching agents in place of the ferric complex salt of EDTA and the ferric complex salt of 1,3-PDTA, which are difficult to biodegrade, is under way. Further, although these metal complex salts are used, besides in bleaching solution compositions, in processing compositions for post-processing, for example, for intensification, reduction, or toning, the problem of biodegradability remains unsolved.

In recent years, as compounds for solving these problems, those described in JP-A-5-72695 ("JP-A" means unexamined published Japanese patent application), JP-A-5-303186, and US-A-5 585 226 have been developed. However, it has been found that use of these compounds brings about new problems. For example, when the ferric complex of ethylenediaminedisuccinic acid (EDDS), i.e. the compound described in JP-A-5-72695 and JP-A-5-303186, or the ferric complex of ethylenediamine-N-carboxymethyl-N'-monosuccinic acid, described in US-A-5 585 226, are used in a bleach-fix solution, there occur such problems as insufficiency of color formation (restoration) and fading (discoloration) due to the bleach-fix solution. To solve the problems, further earnest development efforts are required. Furthermore, ethylenediaminedisuccinic acid (EDDS) and ethylenediamine-N-carboxymethyl-N'-monosuccinic acid are compounds that are not very easily biodegraded, and to decompose them, relatively powerful biodegradation conditions are required. Thus, in view of preservation of the environment, compounds that can be more easily biodegraded have been desired.

EP-A-584665 describes a processing solution for processing an exposed silver halide photographic material comprising a compound of a formula (A) which overlaps with formula (1) of claim 1.

### SUMMARY OF THE INVENTION

Accordingly, the first object of the present invention is to provide a photographic processing composition that exhibits storage stability, and contains aminopolycarboxylic acid-series compounds or iron complex salts thereof, that are excellent in biodegradability.

The second object of the present invention is to provide a processing composition that does not cause problems of photographic properties (e.g. problems of insufficiency of color formation (leuco dye reciprocity failure) due to a bleaching solution, and fading due to a bleach-fix solution), and also to provide a processing method using the same.

The third object of the present invention is to provide a processing method that does not cause precipitation even in an washing bath.

Other and further objects, features, and advantages of the invention will appear more fully from the following description.

### DETAILED DESCRIPTION OF THE INVENTION

As a result of investigation with respect to the above-described problems, the present inventors have found that the above-mentioned objects are attained by a solution containing aminopolycarboxylic acid compounds, or iron

complex salts thereof, shown below, and by a method of processing a silver halide photographic light-sensitive material that comprises using the same. That is, according to the present invention there are provided:

(1) A photographic bleach-fix solution comprising m-carboxybenzene sulfinic acid or p-amino-benzene sulfinic acid and a Fe(III) complex salt of a compound represented by the following formula (I):

formula (I)

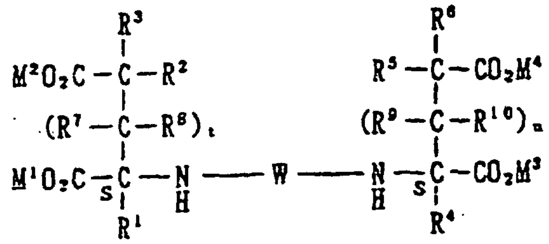

wherein $R^1$, $R^2$, $R^3$, $R^4$, $R^5$, $R^6$, $R^7$, $R^8$, $R^9$, and $R^{10}$ each represent a hydrogen atom, an aliphatic group, an aromatic group, or a hydroxyl group; W represents a divalent linking group containing a carbon atom; $M^1$, $M^2$, $M^3$, and $M^4$ each represent a hydrogen atom or a cation; t and u each represent an integer of 1 to 6; and S indicates that the absolute configuration of an asymmetric carbon is the S-configuration;

(2). The photographic bleach-fix solution as stated above in (1), further comprising a Fe(III) complex salt of ethylenediamine tetraacetic acid.

(3) The photographic bleach-fix solution as stated above in (2), further comprising imidazole.

(4) A method of processing a silver halide color photographic light-sensitive material, comprising processing a silver halide color photographic light-sensitive material exposed to light imagewise with a bleach-fix solution as stated above in (1) to (3).

(5). The method of processing as stated above in (4) wherein the bleaching agent comprises ethylenediaminetetraacetic acid Fe(III) complex salt in an amount of ½ or less in the mole fraction to all the bleaching agent.

The chemical structure of formula (I) is characterized by having two secondary amine structures and four carboxyl groups. Further, the above chemical structure is characterized by at least one methylene chain positioned between the carbon atoms, which carbon atoms each are substituted with the above carboxyl group.

Hitherto known compounds having a similar skeleton are described in JP-A-5-72695 and JT-A-5-303186 (e.g. ethylenediaminedisuccinic acid), as well as in JP-A-7-199433 (e.g. 2-[2-carboxy-2-(1,2-dicarboxyethylamino)-ethylamino]-succinic acid). However, these compounds have not solved the above-mentioned problems as a bleaching agent. Further, similarly compounds described in US-A-5,585,226 (e.g. ethylenediamine-N-carboxymethyl-N'-monosuccinic acid) have not solved the above-mentioned problems. Further, it was difficult to expect that the above-mentioned problems could be solved by the introduction of a group having a carbon chain having not less than carbon atoms of glutaric acid, rather than a succinic acid group, like the compound for use in the present invention. At the present time, the reason why fading is caused by a bleach-fix solution is not clear. Generally, it is believed that the introduction of a long carbon chain is not preferable for the molecular design, because it weakens its coordination force and consequently causes other problems. However, the compound for use in the present invention has solved the above-mentioned problems with no accompanying substantial drawbacks, even though a long carbon chain is introduced.

The compound represented by formula (I) is described in detail below.

The aliphatic group represented by $R^1$, $R^2$, $R^3$, $R^4$, $R^5$, $R^6$, $R^7$, $R^8$, $R^9$, and $R^{10}$ is a straight chain, branched chain, or cyclic alkyl group, alkenyl group, or alkynyl group, each having preferably 1 to 10 carbon atoms. As the aliphatic groups, an alkyl group is preferable, and an alkyl group having 1 to 4 carbon atoms is more preferable, and a methyl group and an ethyl group are particularly preferable.

The aromatic group represented by $R^1$, $R^2$, $R^3$, $R^4$, $R^5$, $R^6$, $R^7$, $R^8$, $R^9$, and $R^{10}$ is a monocyclic or dicyclic aryl group. Examples of the aryl group include a phenyl group and a naphthyl group, with a phenyl group being preferable.

The aliphatic group and the aromatic group each represented by $R^1$, $R^2$, $R^3$, $R^4$, $R^5$, $R^6$, $R^7$, $R^8$, $R^9$, and $R^{10}$ may have a substituent, examples of which include an alkyl group (e.g. methyl, ethyl), an aralkyl group (e.g. phenylmethyl), an alkenyl group (e.g. allyl), an alkynyl group, an alkoxy group (e.g. methoxy, ethoxy), an aryl group (e.g. phenyl, p-methylphenyl), an amino group (e.g. dimethylamino), an acylamino group (e.g. acetylamino), a sulfonylamino group (e.g. methanesulfonylamino), a ureido group, a urethane group, an aryloxy group (e.g. phenyloxy), a sulfamoyl group (e.g. methylsulfamoyl), a carbamoyl group (e.g. carbamoyl, methylcarbamoyl), an alkylthio group (e.g. methylthio), an arylthio group (e.g. phenylthio), a sulfonyl group (e.g. methanesulfonyl), a sulfinyl group (e.g. methanesulfinyl), a hydroxyl group, a halogen atom (e.g. chlorine, bromine, fluorine), a cyano group, a sulfo group, a carboxyl group, a phosphono group, an aryloxycarbonyl group (e.g. phenyloxycarbonyl group), an acyl group (e.g. acetyl, benzoyl), an alkoxycarbonyl group (e.g. methoxycarbonyl), an acyloxy group (e.g. acetoxy), a carbonamido group, a sulfonamido group, a nitro group, and a hydroxamic acid group. Further, the substituent may be a dissociation product or a salt of the above-mentioned group, if possible.

When the above-mentioned substituent contains a carbon atom, the substituent preferably has 1 to 4 carbon atoms.

Preferably, $R^1$, $R^2$, $R^3$, $R^4$, $R^5$, $R^6$, $R^7$, $R^8$, $R^9$, and $R^{10}$ each are a hydrogen atom or a hydroxyl group, with a hydrogen atom being more preferred.

The divalent linking group represented by W can preferably be represented by the following formula (W): formula (W)

$$-(W^1\text{-}D)_m\text{-}(W^2)_n\text{-}$$

wherein $W^1$ and $W^2$, which are the same or different, each represent a straight-chain or branched alkylene group having 2 to 8 carbon atoms (e.g. ethylene, and propylene), a cycloalkylene group having 5 to 10 carbon atoms (e.g. 1,2-cyclohexyl), an arylene group having 6 to 10 carbon atoms (e.g. o-phenylene), an aralkylene group having 7 to 10 carbon atoms (e.g. o-xylenyl), or a carbonyl group. D represents -O-, -S-, $-N(R_W)-$, or a divalent nitrogen-containing heterocyclic group. $R_W$ represents a hydrogen atom or an alkyl group having 1 to 8 carbon atoms (e.g. methyl), or an aryl group having 6 to 10 carbon atoms (e.g. phenyl), each of which may be substituted by $-COOM_a$, $-PO_3M_b$, $M_c$, -OH, or $-SO_3M_d$. $M_a$, $M_b$, $M_c$, and $M_d$, each represent a hydrogen atom or a cation. Examples of the cation include an alkali metal ion (e.g. lithium, sodium, and potassium), an ammonium ion (e.g. ammonium, tetraethylammonium), and a pyridinium ion. The linking group represented by W may be substituted, and examples of the substituent include the substituents that may be possessed by the aliphatic group represented by $R^1$ to $R^{10}$.

As the divalent nitrogen-containing heterocyclic group, a 5- or 6-membered heterocyclic group whose hetero atom is a nitrogen atom, is preferable, and one that is bonded to $W^1$ and $W^2$ at the carbon atoms that are adjacent to each other, such as an imidazolyl group, is more preferable.

$W^1$ and $W^2$ preferably each represent an alkylene group having 2 to 4 carbon atoms.

m is an integer of 0 to 3. When m is 2 or 3, $W^1$-D's are the same or different. m is preferably 0 to 2, more preferably 0 or 1, and particularly preferably 0. n is an integer of 1 to 3. When n is 2 or 3, $W^2$'s are the same or different. n is preferably 1 or 2.

Specific examples of W include, for example, the following:

$-(CH_2)_2-$ , $-(CH_2)_3-$ , $-(CH_2)_4-$ ,

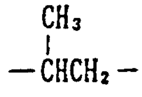 , 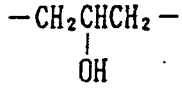 ,

$-CH_2CH_2CH_2OCH_2CH_2-$,

$-CH_2CH_2OCH_2CH_2-$,

$-CH_2CH_2OCH_2CH_2OCH_2CH_2-$,

$-CH_2CH_2SCH_2CH_2-$,

$-CH_2PH_2SCH_2CH_2SCH_2CH_2-$,

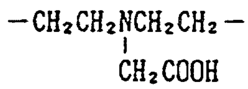 ,

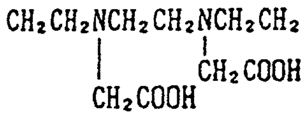 , 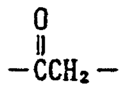 , 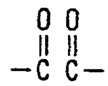 ,

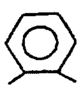 , 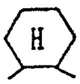 , 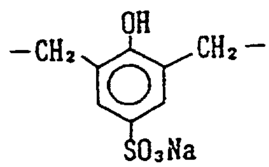 ,

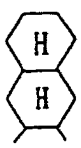 , 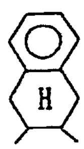 , 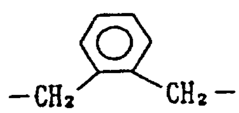 ,

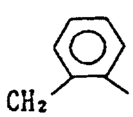 , 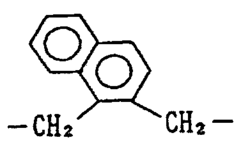 , 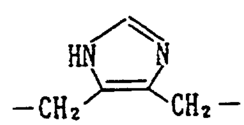 ,

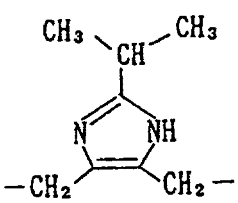 , 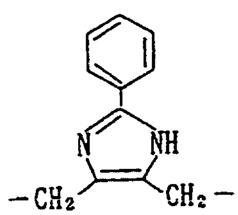 , 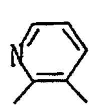 , $-CH_2-CH=CH-CH_2-$ .

Preferably, W is an unsubstituted alkylene group, with an ethylene group and a trimethylene group being more preferred. The ethylene group is most preferred.

Examples of the cation each represented by $M^1$, $M^2$, $M^3$, and $M^4$ include an alkali metal ion (e.g. lithium,

sodium, potassium), an ammonium ion (e.g. ammonium, tetraethylammonium), and a pyridinium ion.

Preferred, of the integer of 1 to 6 represented by t and u, are 1 and 2, with 1 being more preferred. When t and u each are not less than 2, the multiple $R^7$'s are the same or different. This definition is also applied to $R^8$, $R^9$, and $R^{10}$, respectively.

Among the compounds represented by formula (I), those represented by the following formula (II) are preferable, and those represented by the following formula (III) are more preferable.

formula (II)

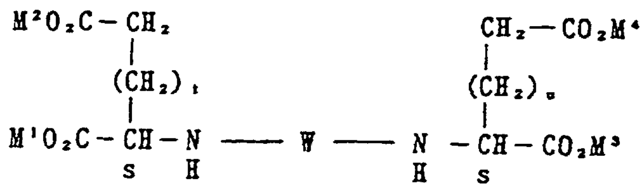

wherein $M^1$, $M^2$, $M^3$, $M^4$, t, u, and W each have the same meanings as those in formula (I), and preferable ones thereof are the same as those preferable in formula (I), and wherein S indicates that the absolute configuration of an asymmetric carbon is the S configuration.

formula (III)

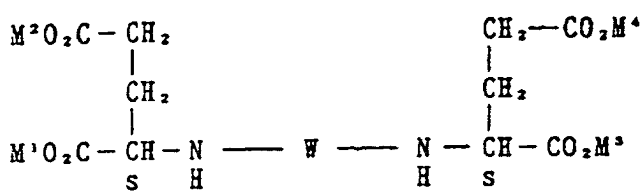

wherein $M^1$, $M^2$, $M^3$, $M^4$, and W each have the same meanings as those in formula (I), and preferable ones thereof are the same as those preferable in formula (I), and wherein S indicates that the absolute configuration of an asymmetric carbon is the S configuration.

Specific examples of the above-mentioned compound represented by formula (I) for use in the present invention are the following.

1. 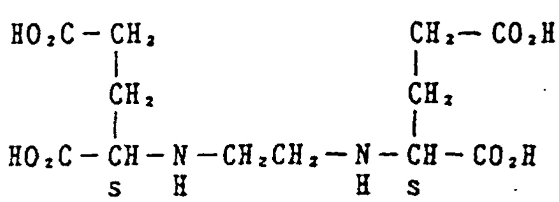

2. 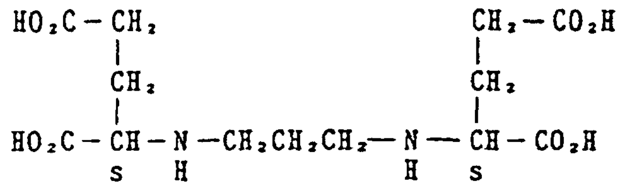

3. 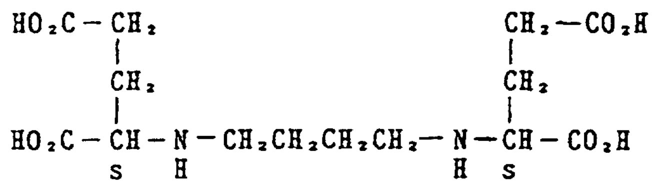

4. 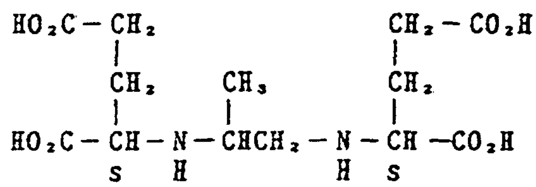

5. 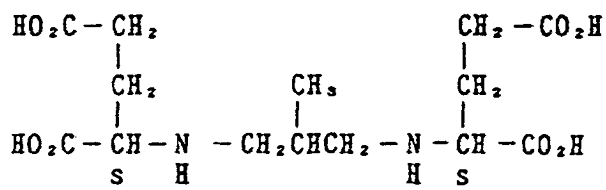

6. 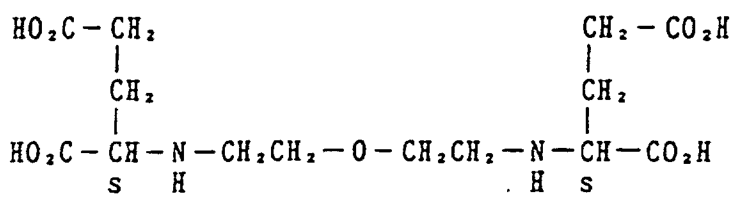

7. 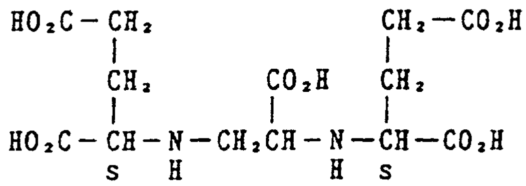

8. 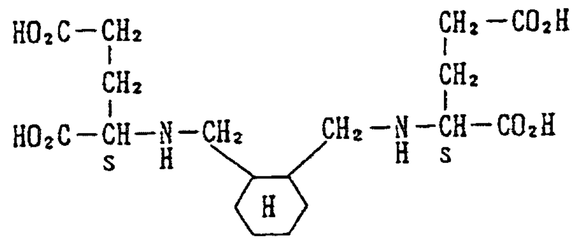

9. 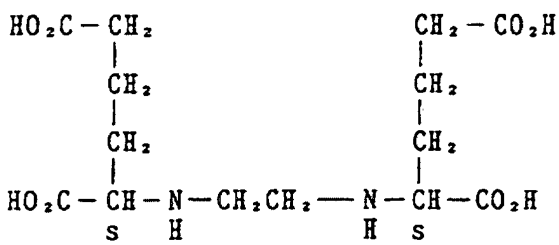

10. 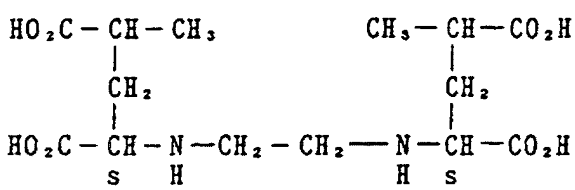

Among the above compounds, the most preferable compound is 1.

The compound represented by formula (I) for use in the present invention can be synthesized in accordance with the descriptions in International Publication Nos. WO 95/12570 and WO 96/35662, JP-A-63-199295, and JP-A-3-173857. These literatures address a synthesis method of the compound represented by formula (I) according to the present invention, and a detergent composition. However, these literatures do not describe that the compound is effective as a photographic chelating agent, and that an Fe(III) complex salt thereof is effective as a bleaching agent for a silver halide color photographic light-sensitive material. Further, it is difficult to find out, in these literatures, whether the compound represented by formula (I), or the Fe(III) complex compound thereof, exhibits biodegradability. Specific examples of the Fe(III) complex salt for use in the present invention are illustrated below.

K − 1

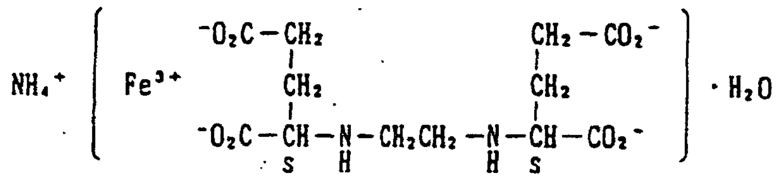

K − 2

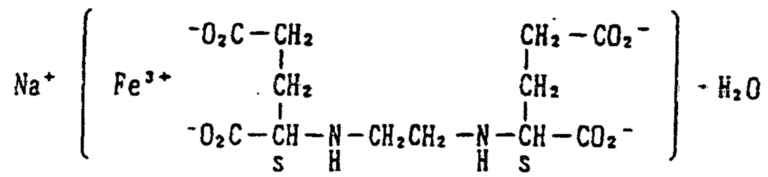

In the present invention, the photographic processing composition is a bleach-fix solution characteristics.

The use of the compound as a bleaching agent is described in detail later. When the compounds is used as a chelating agent or a reducing agent, the compound can be employed in such a content as usually employed as a processing solution containing the same, respectively.

The amount of the compound represented by formula (I) to be added varies depending on a processing composition to which it is added, but generally it is used in an amount of 10 mg to 50 g, per liter of the processing composition.

In more detail, for example, when the compound is added to a black-and-white-developing solution or a color-developing solution, a preferable amount is 0.5 to 10 g, per liter of the processing solution; when it is added to a bleaching solution that comprises hydrogen peroxide, persulfuric acid, bromic acid , a preferable amount is 0.1 to 20 g per liter; when it is added to a fixing solution or a bleach-fix solution, a preferable amount is 1 to 40 g per liter; and when it is added to a stabilizing solution, a preferable amount is 50 mg to 1 g, per liter.

The compound represented by formula (I) may be used alone, or in a combination of at least two kinds of the compound.

Further, the silver halide light-sensitive material for use in the present invention may be not only a color photographic light-sensitive material (e.g. a color negative film, a color photographic printing paper) but also a black and white light-sensitive material (e.g. a light-sensitive material for printing, an X-ray film).

A processing solution having a bleaching capacity for a silver halide color photographic light-sensitive material that is an especially useful application of the present invention, is used in the bleaching step, after the light-sensitive material exposed to light imagewise has been subjected to color-development.

In the present invention, the processing solution (composition) having a bleaching capacity is a bleach-fix solution (composition).

The above-said processing composition may be an aqueous solution, such as a processing solution and a replenisher, each of which is used in a processing.

The ferric complex salt that is used in a processing solution having a bleaching capacity may be added to the processing solution as a preformed ferric complex salt, to dissolve therein, or alternatively a complex-forming compound and a ferric salt (e.g. ferric sulfate, ferric chloride, ferric bromide, Fe(III) nitrate, ammonium Fe(III) sulfate) may be added separately to the processing solution, to coexist them, thereby forming a complex salt in the processing solution having a bleaching capacity.

The complex-forming compound may be used in a little more amount than is necessary to form a complex with a ferric ion. When the complex-forming compound is used in such an excess amount, generally an excess of 0.01 to 10% is preferred.

Preferably, the ferric complex salt-forming compound in a processing solution (composition) having a bleaching capacity according to the present invention, is occupied by not less than 50 mol% of the compound represented by the above-mentioned formula (I), and more preferably not less than 80 mol%.

Further, in the present invention, the above-mentioned compound represented by formula (I), that composes a ferric complex salt-forming compound in a processing solution having a bleaching capacity, may be used alone, or in a combination of at least two kinds thereof.

Further, the ferric complex salt-forming compound in a processing solution having a bleaching capacity may be used in a combination of the above-described compound represented by formula (I) with another compound, as long as the use of such another compound is within the present invention. Examples of the other compound include EDTA, 1,3-PDTA, diethylenetriaminepentaacetic acid, 1,2-cyclohexanediaminetetraacetic acid, iminodiacetic acid, methyliminodiacetic acid, N-(2-acetamido)iminodiacetic acid, nitrilotriacetic acid, N-(2-carboxyethyl)iminodiacetic acid, N-(2-carboxymethyl)iminodipropionic acid, and SS-ethylendiaminedisuccinic acid. However, the other compound is not limited to these compounds in particular.

In the washing bath subsequent to a bleaching bath or a bleach-fix bath, in order to prevent precipitation of iron hydroxide, EDTA and SS-ethylenediaminedisuccinic acid are preferable, with particular preference given to EDTA.

In the present invention, the bleaching agent in a processing solution having a bleaching capacity may be a combination of the above-mentioned ferric complex salt with an inorganic oxidizing agent, as a bleaching agent. Examples of such an inorganic oxidizing agent include hydrogen peroxide, a persulfuric acid salt, and a bromic acid salt. However, the oxidizing agent is not limited to these compounds in particular.

The concentration of the ferric complex salt in a processing solution having a bleaching capacity for use in the present invention is generally in the range of 0.003 to 0.10 mol/l, preferably from 0.02 to 0.50 mol/l, and more preferably from 0.05 to 0.40 mol/l. However, when used with the above-mentioned inorganic oxidizing agent, the concentration of the ferric complex salt is preferably in the range of 0.005 to 0.030 mol/l.

To a processing solution having a bleaching capacity according to the present invention, preferably there are added halides, such as a chloride, a bromide, and an iodide, as a rehalogenizing agent for facilitating oxidation of silver, in addition to containing the said ferric complex salt as a bleaching agent. Alternatively, an organic ligand that is capable of forming a scarcely soluble silver salt may be added thereto, in place of the halides. The halides may be added in the form of a salt thereof, such as an alkali metal salt, an ammonium salt, a guanidine salt, and an amine salt. Specific examples of the salt include sodium bromide, potassium bromide, ammonium bromide, potassium chloride, and guanidine hydrochloride.

Further, a nitric acid salt is preferably used as a corrosion inhibitor, and examples include ammonium nitrate, sodium nitrate, and potassium nitrate. The amount of the nitric acid salt to be added is generally from 0.01 to 2.0 mol/l, and preferably from 0.05 to 0.5 mol/l.

The concentration of a bromide ion in a bleaching solution is preferably not more than 1.8 mol/l, and more preferably from 0.1 to 1.6 mol/l. Further, when used with the above-mentioned inorganic oxidizing agent, the concentration of the bromide ion is preferably from 0.05 to 0.10 mol/l.

The bromide ion may also be added to a bleach-fix solution for use in the present invention, at a preferable concentration range of not more than 1.0 mol/l.

In the present invention, a counter cation to the bromide ion, such as an ammonium ion, a sodium ion, and a potassium ion, may be used. Preferred, of these counter cations, is an ammomonium ion, in view of rapid processing. On the other hand, to maximally stress environmental preservation, preferably there should be substantially no addition of ammounium ion.

The term "substantially no addition of ammonium ion" herein referred to in the present invention, means a concentration of ammonium ion of not more than 0.1 mol/l, preferably not more than 0.08 mol/l, more preferably not more than 0.01 mol/l, and particularly preferably no existence.

In order to control the concentration of an ammonium ion to the above-described range, an alkali metal ion is preferably used as a substitute cation for the ammonium ion, and particularly preferable examples are a sodium ion and a potassium ion. Specific examples include not only a sodium or potassium salt of an aminopolycarboxylic acid ferric complex salt, as a bleaching agent, and potassium bromide and sodium bromide, as a rehalogenizing agent in a bleaching agent, but also potassium nitrate, and sodium nitrate, as a corrosion inhibitor.

Further, preferable examples of an alkali agent for pH adjustment include potassium hydroxide, sodium hydroxide, potassium carbonate, and sodium carbonate.

The pH of a bleaching solution is preferably from 3.0 to 7.0, and particularly preferably from 3.5 to 6.5. On the other hand, the pH of a bleach-fix solution for use in the present invention is preferably from 3.0 to 8.0, more preferably from 4.0 to 7.5, and most preferably from 6.0 to 7.0.

In order to adjust the pH of a processing solution having a bleaching capacity for use in the present invention to the above-mentioned range, a known organic acid may be used therein.

An organic acid having a pKa of 2.0 to 5.5 may be incorporated in a processing solution having a bleaching capacity for use in the present invention, at the concentration of generally from 0.01 to 1.2 mol/l, preferably 0.05 to 0.5 mol/l, and more preferably 0.1 to 0.2 mol/l.

The term "pKa" herein referred to in the present invention means a logarithmic value of the reciprocal of an

acid dissociation constance, that is measured under the conditions of ionic strength 0.1 mol/l at 25 °C.

The organic acid having a pKa of 2.0 to 5.5 for use in the present invention may be a monobasic acid or a polybasic acid. The polybasic acid may be used as an metal salt (e.g. a sodium or potassium salt), or an ammonium salt thereof, as long as the pKa of the polybasic acid is within the above-described range. Further, the organic acid having a pKa within the above-described range may be used as a mixture of at least two kinds of the organic acid.

Preferable specific examples of the organic acid having a pKa of 2.0 to 5.5 for use in the present invention, include various organic acids, for example, aliphatic-series monobasic acids, such as formic acid, acetic acid, monochloroacetic acid, monobromoacetic acid, glycolic acid, propionic acid, monochloropropionic acid, lactic acid, pyruvic acid, acrylic acid, butyric acid, isobutyric acid, pivalic acid, aminobutyric acid, valeric acid, and isovaleric acid; aminoacid-series compounds, such as asparagine, alanine, arginine, ethionine, glycine, glutamine, cysteine, serine, methionine, and leucine; aromatic-series monobasic acids, such as benzoic acid, a monosubstituted benzoic acid (e.g. chlorobenzoic acid, hydroxybenzoic acid), and nicotinic acid; aliphatic-series dibasic acids, such as oxalic acid, malonic acid, succinic acid, tartaric acid, malic acid, maleic acid, fumaric acid, oxalacetic acid, glutaric acid, and adipic acid; aminoacid-series dibasic acids, such as aspartic acid, glutamic acid, cystine, and ascorbic acid; aromatic dibasic acids, such as phthalic acid, and terephtharic acid; and polybasic acids, such as citric acid.

Preferred, of these organic acids for use in the present invention, are acetic acid, glycolic acid, and lactic acid. Acetic acid and glycolic acid are particularly preferred.

The amount of a processing solution having a bleaching capacity to be replenished is generally set in the range of from 20 to 1000 ml, and preferably 40 to 750 ml, per $m^2$ of the light-sensitive material.

Specific example of processing steps having a bleaching capacity are given below.

bleach-fix
bleach/fix
bleach/wash/fix
bleach/bleach-fix
bleach/wash/bleach-fix
bleach/bleach-fix/fix

As the fixing agent contained in a fixing solution or a bleach-fix solution, use can be made of thiosulfates, such as sodium thiosulfate, ammonium thiosulfate, sodium ammonium thiosulfate, and potassium thiosulfate, thiocyanates (rhodanates), such as sodium thiocyanate, ammonium thiocyanate, and potassium thiocyanate, thiourea, and thioethers.

When a thiosulfate is used alone as a fixing agent, the amount of the thiosulfate to be used is generally from 0.3 to 3 mol, and preferably 0.5 to 2 mol, per liter of a fixing solution or a bleach-fix solution. On the other hand, when a thiocyanate is used alone, the amount of the thiocyanate to be used is generally from 1 to 4 mol, per liter of a fixing solution or a bleach-fix solution. The amount of the fixing agent(s) to be used alone or in a combination is generally set in the range of from 0.3 to 5 mol, and preferably from 0.5 to 3.5 mol, per liter of a fixing solution or a bleach-fix solution, with the proviso that when the fixing agents are used in a combination, the total amount thereof is within the above-described range.

Examples of other compounds besides the thiocyanate that can be used in a combination with a thiosulfate, include thiourea and thioethers (e.g. 3,6-dithia-1,8-octanediol).

In a fixing solution, or a bleach-fix solution, can be incorporated a preservative, such as a sulfite (e.g. sodium sulfite, potassium sulfite, ammonium sulfite), hydroxylamine, hydrazine, and an adduct of an acetaldehyde compound and hydrogensulfite (e.g. acetaldehyde sodium hydrogensulfite). Further, various kinds of a fluorescent brightening agent, an antifoaming agent or a surface-active agent, polyvinyl pyrrolidone, or an organic solvent, such as methanol, may be contained therein. The presence of m-carboxybenzene sulfinic acid or p-aminobenzene sulfinic acid is an essential feature of the present invention.

The pH of a fixing solution is preferably from 5 to 9, and more preferably from 6.5 to 8. In order to control the pH of a processing solution having a fixing capacity (a fixing solution, a bleach-fix solution) to the above-described range, a compound having a pKa of from 6 to 9 may also be contained as a buffer in the processing solution. As this compound, imidazoles are preferable, and examples include imidazole, 1-methylimidazole, 1-ethylimidazole, 1-allylimidazole, 1-vinylimidazole, 1-(β-hydroxyethyl)imidazole, 2-methylimidazole, 2-ethylimidazole, 2-amylimidazole, 2-hydroxymethylimidazole, 1-isoamyl-2-methylimidazole, 4-methylimidazole, 4-hydroxymethylimidazole, 4-(β-hydroxyethyl)imidazole, 2,4-dimethylimidazole, 2-ethyl-4-methylimidazole, 4,5-dimethylimidazole, 4-hydroxymethyl-5-methylimidazole, 4-(β-hydroxyethyl)-5-methylimidazole, and 2,4,5-trimethylimidazole. Among the above, imidazole and 2-methylimidazole are preferable, and imidazole is most preferable. These are ones not only excellent in the effect as a buffering agent but also effective in preventing precipitation of iron hydroxide in the subsequent bath. The amount of these compounds to be used is preferably not more than 10 mol, more preferably from 0.01 to 3 mol, and further

preferably 0.05 to 0.5 mol, per liter of the processing solution.

The replenishment rate of the fixing solution is preferably not more than 3000 ml, and more preferably from 200 to 1000 ml, per $m^2$ of the light-sensitive material.

Further, preferably, various kinds of aminopolycarboxylic acids or organic phosphonic acids are added to a fixing solution or a washing water, for stabilization thereof. For example, use can be made of aminotrimethylenephosphonic acid and 1-hydroxyethylidene-1,1-diphosphonic acid.

In the present invention, to the processing solution having a bleaching capacity or its pre-bath, can be added various bleaching accelerators.

Examples of the bleaching accelerator that can be used, include compounds having a mercapto group or a disulfide group, as described in US-A-3 893 858, German patent No. 1 290 812, British patent No. 1 138 842, JP-A-53-95630, and Research Disclosure No. 17129 (July, 1978); thiazolidine derivatives, as described in JP-A-50-140129; thiourea derivatives, as described in US-A-3 706 561; iodides, as described in JP-A-58-16235; polyethylene oxides, as described in German patent No. 2 748 430; polyamine compounds, as described in JP-B-45-8836 ("JP-B" means examined Japanese patent publication); and nitrogen-containing heterocyclic compounds represented by formula (III), as described in JP-A-7-159961. Particularly preferably, the bleaching accelerators are mercapto compounds described in GB-1 138 842.

Further, in the present invention, preferably, the processing time of process having a bleaching capacity is set to a period of time within 4 minutes.

Particularly preferably, the processing solution having a bleaching capacity according to the present invention is subjected to aeration at the time of the processing. Various means known in this technical field can be used for the aeration. For example, can be carried out several methods, such as blowing of air into the processing solution having a bleaching capacity, and absorption of air by means of an ejector.

At the time of the blowing of air, it is preferred to deliver air into a solution through a gas-scattering tube having fine pores. The air-scattering tube is widely used for an airing tub in the activated sludge processing.

For further particulars about the aeration, the articles described in Z-121, Using Process C-41, Third edition (1982), published by Eastman Kodak Co., pp. BL-1 to BL-2, can be referred to.

It is preferred to vigorously stir the processing solution having a bleaching capacity according to the present invention. A method described in JP-A-3-33847, page 8, upper right column, line 6, to the lower left column, line 2, can be used to accomplish the above-mentioned purpose. In particular, the method wherein a jet stream of the bleaching solution is struck against the emulsion surface of the light-sensitive material by using a jet stirring method is preferable.

Further, the processing temperature is not particularly limited, but it is preferably 25 °C to 50 °C, and particularly preferably 35°C to 45°C.

The bleaching solution according to the present invention, can be reused in the processing step by recovering the overflow liquid after use, and then compensating for the composition by the addition of components. Such a usage, which is generally called "regeneration," is preferably used in the present invention. With regard to the details of the regeneration, the items described in Fuji Film Processing Manual, Fuji Color Negative Film, CN-16 Process (revised in August 1990), pp. 39-40 (published by Fuji Photo Film Co., Ltd.), JP-A-3-54555, JP-A-3-48245, and JP-A-3-46653, can be referred to.

The kit for adjusting the bleaching solution according to the present invention may be in the form of a liquid, or a powder, and if ammonium salts are eliminated, most of the raw materials can be supplied in the form of powders, and materials that are less hydroscopic more easily form a powder.

Preferably the above kit for the regeneration is preferably in the form of a powder, in view of the reduction of the amount of waste liquor, because in that case, excess water is not used and it can be directly used.

With regard to the regeneration of the bleaching solution, in addition to the above described aeration methods, the methods disclosed in Shashin Kogaku no Kiso - Ginn-en Shashin Hen (The Fundamentals of Photographic Technology-Silver Salt Photography) (edited by Nippon Shashin Gakkai, published by Corona, Co., 1979), can be utilized. Specific examples of the regeneration methods of the solution include a regeneration method of a bleaching solution by electrolysis and a regeneration method by a hydrogen peroxide, a bromous acid, ozone making use of a bromic acid, a chlorous acid, a bromine, a bromine precursor, a persulfate, a hydrogen peroxide, and a catalyst, but the present invention is not limited to them.

In the regeneration method by electrolysis, a regeneration processing is carried out by putting an anode and a cathode in the same bleaching bath, or by separating an anode bath from a cathode bath by a diaphragm, as well as that a bleaching solution and a developing solution and/or a fixing solution can be regeneration-processed at the same time, also using a diaphragm.

Preferably, a color-developing solution for use in the present invention is one described in JP-A-3-33847, page 9, left upper column, line 6, to page 11, right under column, line 6.

Specifically, color developing solutions and their replenishers of CN-16, CN-16X, CN-16Q, and CN-16FA (trade names), all of which are processing agents for a color negative film, manufactured by Fuji Photo Film Co., Ltd.,

or color developing solutions of C-41, C-41B, and C-41RA (trade names), all of which are processing agents for a color negative film, manufactured by Eastman Kodak Co., Ltd., are preferably used.

Further, the color-developing solution for use in the present invention can be regenerated by the regeneration methods described in JP-A-3-95552 and JP-A-3-95553.

In the fixing or bleach-fix step, as well as the bleaching step, a strengthened stirring is preferred. Specifically, the above-mentioned jet stirring method is most preferred.

Further, silver is removed from a fixing solution and a bleach-fix solution by a known method; this reduces the replenisher volume and enables recycling use.

With respect to the washing step and the stabilizing step that is carried out in the present invention, the items described in JP-A-3-33847, page 11, right under column, line 9, to page 12, right upper column, line 19, can be preferably performed.

Formaldehyde has been conventionally used as a stabilizing agent in a stabilizing solution. However, stabilizers, such as N-methylolpyrazol, hexamethylenetetramine, an adduct of formaldehyde and hydrogensulfurous acid, dimethylolurea, and trizazole derivatives (e.g. 1,4-4-bis(1,2,4-triazole-1-iminomethyl)piperazine), are preferably used, from a viewpoint of working environmental safety. Most of all, the use of N-methylolpyrazole that is obtained by reacting formaldehyde with pyrazole, or a combination of triazoles, such as 1,2,4-triazole, and triazole derivatives, such as 1,4-4-bis(1,2,4-triazole-1-iminomethyl)piperazine (Japanese patent application No. 3-159918), is preferred, on the grounds that image stability is high, while formaldehyde vapor pressure is low.

Further, the washing water can contain a variety of bacteria-proofing agents and mildew-proofing agents, to prevent the formation of scale or the existence of mildew occurring on processed light-sensitive materials. As the bacteria-proofing agents and mildew-proofing agents, can be used, for example, one or more of thiazorilbenzimidazole compounds, as described in JP-A-57-157244 and JP-A-58-105145, isothiazolone compounds, as described.in JP-A-57-8542, and general-purpose mildew-proofing agents described in "Journal Antibacteria and Antifungus Agents," Vol. 1, No. 5, pp 207 to 223 (1983), such as chlorophenol compounds, represented by trichlorophenol; bromophenol compounds, organotin compounds, organozinc compounds, thiocyanic acid compounds, isothiocyanic acid compounds, acid amide compounds, diazine compounds, triazine compounds, thiourea compounds, benzotriazolealkylguanidine compounds, quaternary ammonium salts, represented by benzalkonium chloride; or antibiotics, represented by penicillins. Further, various fungicides described in JP-A-48-83820 can be used.

Further, preferably, various chelating agents are additionally used. Preferable compounds as the chelating agents include, for example, aminopolycarboxylic acids, such as ethylenediaminetetraacetic acid and diethylenetriaminepentaacetic acid, organic phosphonic acids, such as 1-hydroxyethylidene-1,1-diphosphonic acid and diethylenetriamine-N,N,N',N'-tetramethylenephosphonic acid, and hydrolyzates of maleic anhydride polymers described in EP-A-345 172 (A1).

In particular, the present invention can be effectively applied to a bleach processing method for various kinds of a color photographic light-sensitive material, such as a color negative film, a color reversal film, a color paper, a color reversal paper, a cinematographic color negative film, and a cinematographic color positive film. For example, preferable light-sensitive materials are those described in JP-A-3-33847, page 12, right upper column, line 29, to page 17, right upper column, line 17, and JP-A-4-73748.

In particular, a light-sensitive material having a dried film thickness of not more than 20 μm is preferred, because of excellent bleaching. In particularly, a light-sensitive material having a dried film thickness of not more than 18 μm is preferred.

Further, a light-sensitive material that exhibits a high swelling rate is preferred. Specifically, those described in the above-described JP-A-3-33847, page 14, left upper column, lines 7 to 14, are particularly preferred.

A processing solution having a bleaching capacity according to the present invention may contain not only an Fe(III) complex salt of the compound represented by the above-described formula (I), but also an Mn(III), Co (III), Rh(II), Rh(III), Au(II), Au(III), or Ce(IV) complex salt of the compound represented by formula (I).

Further, these heavy metal complex salts including an Fe(III) complex salt may be used not only for a bleach processing composition but also for such a processing composition used in the post-treatment of black and white films as a an intensifier, a reduction solution, and a toner.

According to the present invention, stabilization of a processing composition, reduction of fading due to a bleach-fix solution, prevention of precipitation in a water bath, and furthermore processing with less environmental pollution, were attained.

The present invention will be described in more detail with reference to examples, but the present invention is not restricted to them.

EXAMPLES

Example 1

Sample 101, which was a photographing multi-layer color light-sensitive material (color negative film) described in Example 1 of JP-A-5-303186, was prepared.
Sample 101 was cut to a width of 35 mm, it was exposed imagewise, and it was continuously processed with the below-shown processing solutions, until the replenishing rate of the. bleach-fix solution reached 10 times the tank volume.
The bleach-fix solution was passed through a silver recovering apparatus in an in-line manner, to recover silver. Part of the overflow from the silver recovering apparatus was discharged as waste liquor, and the remainder was regenerated and was reused as a replenisher of the bleach-fix solution. The silver recovering apparatus was a small-sized electrolytic silver recovering apparatus, wherein the anode was made of carbon and the cathode was made of stainless steel, and the current density used was 0.5 A/$dm^2$. A schematic view of the system for recovering silver is illustrated in Fig. 1 of JP-A-6-175305. That is, an overflow 21 of a bleach-fix tank 20 was connected directly to the silver recovering apparatus 22, and part of the overflow, in an amount of 100 ml per 1 min, was returned to the original bleach-fix tank 20, by a pump 23 through a filter 24. An overflow 25 from the silver recovering apparatus 22 was recovered, in an amount of 300 ml per liter of the overflow, to a regeneration tank 26. When the recovered amount reached 1 liter, air was bubbled there-through for about 2 hours; then a regenerator 28 was added, and the resulting solution was sent to a replenishing tank 30 of the bleach-fix solution by a pump 29. The remainder (100 ml) was discharged at 27 as waste liquor. The amount of the waste liquor was 220 ml per $m^2$ processing of Sample 101.
The washing process was carried out using 5-stage multi-chamber water washing tanks arranged side by side in a countercurrent cascade manner. Specifically, use was made of one shown in Fig. 1 of JP-A-5-66540.
The overflow of the first washing water $W_1$ was cascaded to the previous bleach-fix tank. A reverse osmosis membrane (RO) apparatus RC 30 (trade name, manufactured by Fuji Photo Film Co., Ltd.) was placed between the fourth washing $W_4$ and the fifth washing $W_5$. Namely, washing water taken from $W_4$ was passed through the RO apparatus, the condensed solution was returned to $W_4$, and the passed solution was returned to $W_4$. A schematic diagram of the processor is shown in Fig. 2 of JP-A-6-175305. The processing step ($N_{Blix}$) is shown below.

| Processing step ($N_{Blix}$) | | | | |
|---|---|---|---|---|
| Processing step | Processing | | Replenishing rate*1 | Tank Volume /liter |
| | time | temperature | | |
| Color developing | 2 min 00 sec | 45 °C | 104 ml | 2 |
| Bleach-Fixing | 2 min 30 sec | 45 °C | 200 ml | 2 |
| Washing (1) | 15 sec | 45 °C | - | 0.5 |
| washing (2) | 15 sec | 45 °C | - | 0.5 |
| Washing (3) | 15 sec | 45 °C | - | 0.5 |
| Washing (4) | 15 sec | 45 °C | - | 0.5 |
| Washing (5) | 15 sec | 45 °C | 104 ml | 0.5 |
| Stabilizing | 2 sec | Room temperature | 30 ml | Coated |
| Drying | 50 sec | 70 °C | - | - |

*1 the replenishment rate was the amount per $m^2$ of the light-sensitive material

The crossover time from the color development to the bleach fix, and from the bleach-fix to the washing (1), each was 3 sec. This crossover time was included in the processing time in the preceding bath. Moreover, the average amount carried over per $m^2$ of the light-sensitive material was 65 ml.
The temperature and relative humidity of the atmosphere around each processor were detected by a hygrothermal meter, as shown in JP-A-3-280042, to calculate the evaporated amount, and the evaporation was compensated for each tank. As the water for the compensation, deionized water for the above washing water was used.
The composition of the processing solutions is shown below:

| (Color-developer) | Mother Solution (g) | Replenisher (g) |
|---|---|---|
| Diethylenetriaminepentaacetic acid | 1.2 | 4.0 |
| 1-Hydroxyethylidene-1,1-diphosphonic acid | 2.7 | 3.3 |

(continued)

| (Color-developer) | Mother Solution (g) | Replenisher (g) |
|---|---|---|
| Potassium hydroxide | 2.50 | 3.90 |
| Sodium sulfite | 3.84 | 9.0 |
| Sodium bicarbonate | 1.8 | - |
| Potassium carbonate | 31.7 | 39.0 |
| Potassium bromide | 5.60 | - |
| Potassium iodide | 1.3 mg | - |
| Hydroxylamine sulfate | 2.5 | 6.9 |
| 2-Methyl-4-[N-ethyl-N-(β-hydroxyethyl)amino]aniline sulfate | 9.0 | 18.5 |
| Water to make | 1.0 liter | 1.0 liter |
| pH | 10.05 | 11.90 |

| (Bleach-Fixing solution) | Mother solution (mol) | Replenisher at start (mol) |
|---|---|---|
| Ammonium thiosulfate | 1.4 | 2.31 |
| Chelating agent described in Table 1 | 0.17 | 0.28 |
| Ferric nitrate nonahydrate | 0.15 | 0.25 |
| Ammonium bisulfate | 0.10 | 0.17 |
| m-Carboxybenzenesulfinic acid | 0.05 | 0.09 |
| Water to make | 1.0 liter | 1.0 liter |
| pH (25 °C) | 6.0 | 6.0 |
| (pH was adjusted by acetic acid and aqueous ammonia) | | |

| (Bleach-Fixing regenerating agent) (Amount added per liter of the recovered solution for regeneration (g)) | |
|---|---|
| Ammonium thiosulfate | 0.91 |
| Chelating agent described in Table 1 | 0.11 |
| Ferric nitrate nonahydrate | 0.10 |
| Ammonium sulfite | 0.07 |
| m-Carboxybenzenesulfinic acid | 0.04 |

(Washing water) (Both mother solution and replenisher)

Tap water was treated by passage through a mixed bed ion-exchange column filled with H-type strong acidic cation exchange resin (Amberlite IR-120B, trade name, made by Rohm & Haas) and OH-type strong basic anion exchange resin (Amberlite IRA-400, the same as the above) so that the concentrations of Ca ions and Mg ions in water were both made to decrease to 3 mg/liter or below, followed by adding 20 mg/liter of sodium dichlorinated isocyanurate and 150 mg/liter of sodium sulfate. The pH of this water was in the range of 6.5 to 7.5.

| (Stabilizing solution) for coating | (g) |
|---|---|
| Formalin (37%) | 2.0 ml |
| Polyoxyethylene-p-monononylphenylether (av. polymerization degree: 10) | 0.3 |
| Disodium ethylenediaminetetraacetate | 0.05 |
| Water to make | 1.0 liter |
| pH | 5.0 to 8.0 |

The above processing system was carried out, and the amount of the waste liquor was 22 liters after 100 $m^2$ of Sample 101 was processed.

The amount of residual silver in the maximum density section of the multi-layer color light-sensitive material

Sample 101 subjected to the above processing was measured by fluorescent X-ray analysis. The result is shown in Table 1. The $D_{min}$ values of these samples obtained by processing in this manner were measured using green light (G light) to be read, respectively.

Then, the bleach-fix step of the above processing was changed to four steps, bleach-washing (A)-washing (B)-fixing, as a reference processing method free from bleach fogging, and the processing was carried out using the below-shown processing solution formulation. There was no change, except for the below-shown changes.

| Processing step | Processing | | Replenisher rate |
|---|---|---|---|
| | time | temperature | |
| Bleaching | 3 min 00 sec | 38 °C | 710 ml |
| Washing (A) | 15 sec | 24 °C | Countercurrent piping system from (B) to (A) |
| Washing (B) | 15 sec | 24 °C | 430 ml |
| Stabilizing | 3 min 00 sec | 38 °C | 430 ml |

| (Basic bleaching solution) | Tank solution (g) | Replenisher (g) |
|---|---|---|
| Ethylenediaminetetraacetic acid ferric complex salt sodium trihydrate | 100.0 | 120.0 |
| Disodium ethylenediaminetetraacetate | 10.0 | 11.0 |
| 3-Mercapto-1,2,4-triazole | 0.03 | 0.08 |
| Ammonium bromide | 140.0 | 160.0 |
| Ammonium nitrate | 30.0 | 35.0 |
| Aqueous ammonia (27%) | 6.5 ml | 4.0 ml |
| Water to make | 1.0 liter | 1.0 liter |
| pH | 6.0 | 5.7 |
| (pH was adjusted by aqueous ammonia and nitric acid) | | |

| (Fixing solution) | Tank solution (g) | Replenisher (g) |
|---|---|---|
| Disodium ethylenediaminetetraacetate | 0.5 | 0.7 |
| Ammonium sulfite | 20.0 | 22.0 |
| Aqueous ammonium thiosulfate solution (700 g/liter) | 295.0 ml | 320.0 ml |
| Acetic acid (90%) | 3.3 | 4.0 |
| Water to make | 1.0 liter | 1.0 liter |
| pH | 6.7 | 6.8 |
| (pH was adjusted by aqueous ammonia and acetic acid) | | |

Similarly, the Dmin values of the processed light-sensitive materials obtained by using the above reference bleaching solution were read. The differences, the ΔDmin, between these Dmin values of the light-sensitive materials and the Dmin of the reference bleaching solution as a reference, were found. In passing, the Dmin value obtained by using the reference bleaching solution was 0.60.

Bleach fog (ΔDmin) = (Dmin of each sample) -

(Dmin of the reference bleaching solution)

Then, using the above Sample 101, the increase in stain during storage of the processed light-sensitive material was found, from a change in density of Dmin of the color-unformed section between those before and after storage under the following conditions.

Dark/heat and humidity conditions: 60 °C, 70% RH, 4 weeks

Stain increase (ΔD) = (Dmin after storage) -

(Dmin before storage)

To investigate the degree of insufficiency of color formation, Sample 101 processed in the processing step ($N_{Blix}$) was processed in the below processing step ($N_{BL}$) again.

| Processing step ($N_{BL}$) | | | |
|---|---|---|---|
| Processing step | Processing | | Replenishing rate*1 |
| | time | temperature | |
| Bleaching | 3 min 00 sec | 38 °C | 710 ml |
| Washing (C) | 15 sec | 24 °C | Countercurrent piping system from (D) to (C) |
| Washing (D) | 15 sec | 24 °C | 430 ml |
| Stabilizing | 3 min 00 sec | 38 °C | 430 ml |
| Washing (6) | 15 sec | 45 °C | - |
| Washing (7) | 15 sec | 45 °C | - |
| Washing (8) | 15 sec | 45 °C | - |
| Washing (9) | 15 sec | 45 °C | - |
| Washing (10) | 15 sec | 45 °C | 104 ml |
| Stabilizing | 2 sec | Room temperature | 30 ml |
| Drying | 50 sec | 70 °C | - |

*1 the replenishment rate was the amount per $m^2$ of the light-sensitive material

For the bleaching, washing, fixing, and stabilizing, the above reference bleaching solution, washing water, fixing solution, and stabilizing solution were used, respectively.

The Dmax values of Sample 101, measured with red light (R light) before and after processed in the above processing step ($N_{BL}$), were read.

Insufficiency of color formation (ΔDmax) = (Dmax after processed in $N_{BL}$) - (Dmax of each sample)

The chelating agents used in the bleach-fix solution and the bleach-fix regenerator were respectively changed to those shown in Table 1, and tests of Nos. 101 to 109 were carried out.

The results of the above evaluation are shown in Table 1.

Table 1

| No. | Chelating agent | Amount of residual silver $\mu g/cm^2$ | Bleach fogging $\Delta Dmin$ (G) | Stain increase $\Delta D$(G) | Insufficiency of color formation $\Delta Dmax$ (R) | Remarks |
|---|---|---|---|---|---|---|
| 101 | Comparative compound A | 16.5 | 0.00 | 0.01 | 0.00 | Comparative example |
| 102 | Comparative compound B | 11.7 | 0.08 | 0.08 | 0.00 | Comparative example |
| 103 | Comparative compound C | 6.2 | 0.00 | 0.01 | 0.20 | Comparative example |
| 104 | Comparative compound D | 5.8 | 0.01 | 0.01 | 0.18 | Comparative example |
| 105 | Comparative compound E | 7.1 | 0.00 | 0.01 | 0.20 | Comparative example |
| 106 | Exemplified compound 1 | 3.0 | 0.00 | 0.01 | 0.00 | This invention |
| 107 | Exemplified compound 2 | 5.3 | 0.00 | 0.01 | 0.01 | This invention |
| 108 | Exemplified compound 7 | 5.8 | 0.00 | 0.01 | 0.01 | This invention |
| 109 | Exemplified compound 9 | 5.8 | 0.00 | 0.01 | 0.02 | This invention |

Comparative compound A

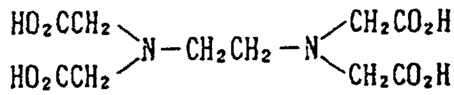

EDTA

Comparative compound B

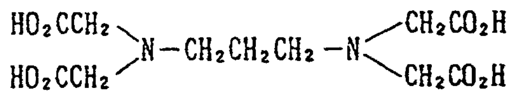

Comparative compound C

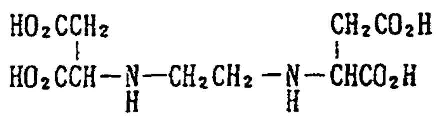

EDDS

Comparative compound D

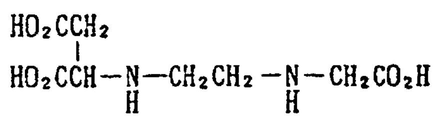

EDMS

Comparative compound E

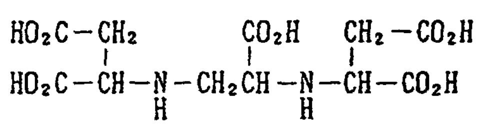

From the results shown in Table 1, the superiority of the present invention on the improvement of desilvering, bleach fogging, stain increase, and insufficiency of color formation is comprehensively and satisfactorily apparent.

Moreover, as Comparative compound A, one labeled DOJINDO manufactured by Wako Pure Chemical Industries, Ltd. was used. As Comparative compound B, a white powder obtained by reacting 1,3-propanediamine and chloroacetic acid in an aqueous solvent in the presence of an alkali and rendering the resultant product acidic (pH 2) with hydrochloric acid, was used. As Comparative compound C, one synthesized by the method described in US-A-3 158 635, was used.

Use was made, as Comparative compound D, a white powder obtained by reacting ethyleneimine and chloroacetic acid in an aqueous solvent in the presence of sodium hydroxide, adding DL-aspartic acid thereto, and heating the resultant mixture for 3 hours, followed by rendering the resultant product acidic (pH 2) with hydrochloric acid. According to this method, using L-aspartic acid, EDMS in the S-form can also be synthesized.

As Comparative compound E, one synthesized by the method described in JP-A-7-199433, was used.

Example 2

A multi-layered color photographic printing paper (sample 001) according to Example 4 in JP-A-5-303186 and the following processing solutions were prepared.

| (Color-developer) | Tank Solution | Reple-nisher |
|---|---|---|
| Cation-exchanged water | 800.0ml | 800.0 ml |
| Compound M | 0.1 g | 0.1 g |

Compound M

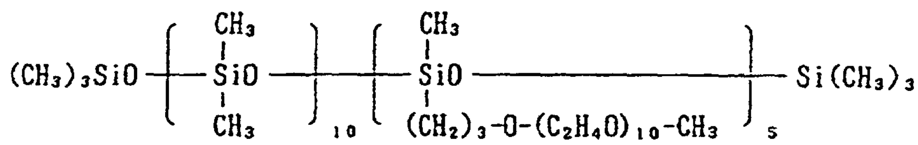

| | | |
|---|---|---|
| Triisopropanolamine | 15.0 g | 15.0 g |
| Potassium hydroxide | 3.0 g | 3.0 g |
| Ethylenediaminetetraacetic acid | 4.0 g | 4.0 g |
| Sodium 4,5-dihydroxybenzene -1,3-disulfonate | 0.5 g | 0.5 g |
| Potassium chloride | 14.5 g | - |
| Potassium bromide | 0.04 g | - |
| Fluorescent whitening agent (Compound N) | 2.5 g | 3.0 g |

Compound N

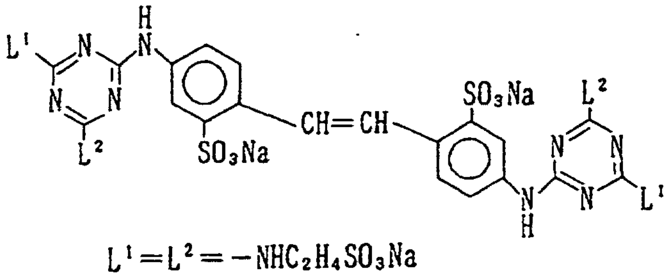

$L^1=L^2=-NHC_2H_4SO_3Na$

| | | |
|---|---|---|
| Sodium sulfite | 0.1 g | 0.1 g |
| Disodium-N,N-bis(sulfonatoethyl)hydroxylamine | 8.5 g | 11.0 g |
| N-Ethyl-N-(β-methanesulfonamidoethyl)-3-methyl-4-aminoaniline •3/2 sulfuric acid•monohydrate | 5.0 g | 11.5 g |
| Potassium carbonate | 26.3 g | 26.3 g |
| Water to make | 1000.0 ml | 1000.0 ml |
| pH | 10.15 | 11.15 |
| (at 25 °C / pH was adjusted by KOH or sulfuric acid) | | |

| (Bleach-Fix solution) | | |
|---|---|---|
| Water | 700.0 ml | 700.0 ml |
| Ammonium thiosulfate (750 g/liter) | 100.0 ml | 250.0 ml |
| Ammonium sulfite | 35.0 g | 88.0 g |
| p-Aminobenzenesulfinic acid | 5.0 g | 12.5 g |
| Imidazole | 8.0 g | 20.0 g |
| Chelating agent (shown in Table 2) | 0.11 mol | 0.28 mol |
| Ferric nitrate nonahydrate | 40.4 g | 101 g |
| Water to make | 1000.0 ml | 1000.0 ml |
| pH | 7.0 | 6.8 |
| (at 25 °C / pH was adjusted by nitric acid or aqueous ammonia) | | |

| (Rinse) Common to (1) to (4) | | |
|---|---|---|
| Sodium chlorinated isocyanurate | 0.02 g | 0.02 g |
| Deionized water (conductivity: 5 μS/cm or below) | 1000.0 ml | 1000.0 ml |
| pH | 6.5 | 6.5 |

| [Processing step ($P_{Blix}$)] | | | | |
|---|---|---|---|---|
| Processing step | Processing | | Replenishing rate * | Tank Volume |
| | temperature | time | | |
| Color developing | 39 °C | 45 sec | 70 ml | 20 liter |
| Bleach-Fixing | 35 °C | 45 sec | 60 ml** | 20 liter |
| Rinse (1) | 35 °C | 20 sec | - | 10 liter |
| Rinse (2) | 35 °C | 20 sec | - | 10 liter |
| Rinse (3) | 35 °C | 20 sec | 360 ml | 10 liter |
| Drying | 80 °C | 60 sec | | |

(* the replenishment rate was the amount per $m^2$ of the light-sensitive material)
(the rinse was conducted in a 3-tank counter-current system of Rinse (3) → (1))
(** in addition to the above 60 ml, 120 ml per $m^2$ of the light-sensitive material was poured from Rinse (1))

To investigate the amount of residual silver after the processing, the multi-layer color photographic printing paper (Sample 001) was exposed uniformly to light, so that the gray density would be 2.2, and then it was processed in the above processing step ($P_{Blix}$). The amount of residual silver was measured by fluorescent X-ray analysis.

To examine the Blix fading, Sample 001, which was subjected to gradation exposure through a wedge and then was processed in the processing step $P_{Blix}$, was processed again in the below reprocessing step $P_{EDTA}$. By comparing the Dmax values measured with red light (R light) before and after the reprocessing, the extent of the Blix fading was examined.

| [Reprocessing step $P_{EDTA}$] | | | | |
|---|---|---|---|---|
| | Processing | | Replenishing rate* | |
| Processing step | temperature | time | | Tank Volume |
| Bleach-Fixing | 35 °C | 45 sec | 60 ml** | 20 liter |
| Rinse (4) | 35 °C | 20 sec | - | 10 liter |
| Rinse (5) | 35 °C | 20 sec | - | 10 liter |
| Rinse (6) | 35 °C | 20 sec | 360 ml | 10 liter |
| Drying | 35 °C | 60 sec | | |

(* the replenishment rate was the amount per $m^2$ of the light-sensitive material)
(the rinse was conducted in a 3-tank counter-current system of Rinse (6) → (4))
(** in addition to the above 60 ml, 120 ml per $m^2$ of the light-sensitive material was poured from Rinse (4))

The process ($P_{Blix}$) was started by using the above processing solutions and placing the tank solutions in respective processing tanks in the above processing step $P_{Blix}$, and it was continued by adding the replenishers to the respective tanks in accordance with the processed amount. The process was carried out until the accumulated replenishment rate reached three times the tank volume. The results of the processing carried out at this point are shown in Table 2. The reprocess ($P_{EDTA}$) was carried out in the above reprocessing step $P_{EDTA}$ with the chelating agent of the bleach-fix solution changed to ethylenediaminetetraacetic acid. Other components were the same as those in $P_{Blix}$, provided that the reprocess ($P_{EDTA}$) was carried out with the respective solutions being fresh.

Tests of Nos. 201 to 209 were carried out with the chelating agent used in the bleach-fix solution changed to those shown in Table 2, respectively.

The results are shown in Table 2.

Table 2

| No. | Chelating agent | Amount of residual silver µg/cm$^2$ pH 7.0 | Blix fading ΔDmax(R) pH 7.0 | Remarks |
|---|---|---|---|---|
| 201 | Comparative compound A | 2.6 | 0.00 | Comparative example |
| 202 | Comparative compound B | 10.6 | 0.00 | Comparative example |
| 203 | Comparative compound C | 1.8 | 0.19 | Comparative example |
| 204 | Comparative compound D | 1.5 | 0.17 | Comparative example |
| 205 | Comparative compound E | 1.9 | 0.19 | Comparative example |
| 206 | Exemplified compound 1 | 0.9 | 0.00 | This invention |
| 207 | Exemplified compound 2 | 1.7 | 0.01 | This invention |
| 208 | Exemplified compound 7 | 1.9 | 0.01 | This invention |
| 209 | Exemplified compound 9 | 1.8 | 0.02 | This invention |

Comparative compounds A, B, C, D, and E were the same as those in Example 1.

From the results shown in Table 2, the superiority of the present invention is apparent as compared to the comparative examples in view of the desilvering, and the Blix fading (fading due to the bleach-fix solution).

Example 3

Sample 301 of a multi-layer color light-sensitive material was prepared in the same manner as in Example 1 of JP-A-5-165176, except that, instead of the cellulose triacetate film base having an undercoat layer used in the multi-layer color light-sensitive material A prepared in Example 1 of JP-A-5-165176, a polyethylene naphthalate base having a thickness of 100 µm was used, and the backing surface of the polyethylene naphthalate base was coated with a striped magnetic recording layer described in Example 1 of JP-A-4-124628. This Sample 301 was subjected to the same test as that for Nos. 101 to 109 of Example 1 of this application, and, similarly to Example 1 of this application, the effects of the present invention were obtained.

Further, Sample 302 of a multi-layer color light-sensitive material was prepared in the same manner as in Example 1 of this application, except that, instead of the base used in the multi-layer color light-sensitive material 101 of Example 1 of this application, the base and the backing layer in Sample No. 1-3 in Example 1 of JP-A-4-62543 were used, and the second protective layer was coated with $C_8F_{17}SO_2N(C_3H_7)CH_2COOK$, in an amount of 15 mg/m$^2$. The thus-prepared Sample 302 was worked into a format shown in Fig. 5 of JP-A-4-62543 and was tested in the same manner as for Nos. 101 to 109 of Example 1 of this application, and, similarly to Example 1 of this application, the effects of the present invention were obtained.

Example 4

Sample 702 of the below-shown multi-layer color light-sensitive material was processed, using the processing steps and processing solutions described in Example 1 of this application, with a cine-type automatic processor, and the same evaluation as in Example 1 of this application was effected.

(1) Material of the base

The base used in this Example was prepared by the following method:

PEN: 100 parts by weight of a commercially available poly(ethylene-2,6-naphthalate) polymer, and 2 parts by weight of Tinuvin P.326 (trade name, manufactured by Ciba-Geigy), as an ultraviolet absorber, were dried in a usual manner, melted at 300 °C, and extruded from a T-die, and the extrudate was stretched longitudinally 3.3-folds at 140 °C; then it was stretched laterally 3.3-folds at 130 °C, and it was subjected to heat setting for 6 sec at 250 °C. The glass transition temperature of the product was 120 °C.

(2) Application of an undercoat layer

Both surfaces of the above base were subjected to corona discharge treatment, and an undercoat liquid having the below-shown composition was applied, so that an undercoat layer would be provided on the higher-temperature surface during the stretching of the base. The corona discharge treatment was carried out using a solid-state corona treatment apparatus 6KVA model, manufactured by Pillar Inc., and the base, of width 30 cm, was treated at 20 m/min. The subject was treated at 0.375 KV.A.min/m$^2$, which was confirmed from the readings of the electric current and the voltage. The discharge frequency at the treatment was 9.6 KHz, and the gap clearance between the electrode and the dielectric roll was 1.6 mm.

| | |
|---|---|
| Gelatin | 3 g |
| Distilled water | 250 ml |
| Sodium-α-sulfodi-2-ethylhexylsuccinate | 0.05 g |
| Formaldehyde | 0.02 g |

Further, a undercoat layer having the following composition was provided to a TAC support.

| | |
|---|---|
| Gelatin | 0.2 g |
| Salicylic acid | 0.1 g |
| Methanol | 15 ml |
| Acetone | 85 ml |
| Formaldehyde | 0.01 g |

(3) Application of backing layers

The following first to third backing layers were applied to one side of the base having the undercoat made in the above (2).

a) First backing layer

| | |
|---|---|
| Co-containing needle-like γ-iron oxide fine particles (having an average particle diameter of 0.08 μm and contained as a dispersion in gelatin) | 0.2 g/m$^2$ |
| Gelatin | 3 g/m$^2$ |
| Compound shown below | 0.1 g/m$^2$ |

$$(CH_2{=}CHSO_2NHCH_2CH_2NH)_2CO$$

| | |
|---|---|
| Compound shown below | 0.02 g/m$^2$ |

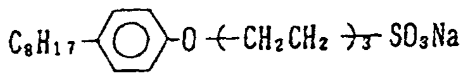

| | |
|---|---|
| Poly(ethyl acrylate) (having an average diameter of 0.08 μm) | 1 $g/m^2$ |

b) Second backing layer

| | |
|---|---|
| Gelatin | 0.05 $g/m^2$ |
| Electrically conductive material [$SnO_2/Sb_2O_3$ (9 : 1), grain diameter 0.15 μm] | 0.16 $mg/m^2$ |
| Sodium dodecylbenzenesulfonate | 0.05 $g/m^2$ |

c) Third backing layer

| | |
|---|---|
| Gelatin | 0.5 $g/m^2$ |
| Poly(methyl methacylate) (average grain diameter 1.5 μm) | 0.02 $g/m^2$ |
| Cetyl stearate (dispersion with sodium dodecylbenzenesulfonate) | 0.01 $g/m^2$ |
| Sodium di(2-ethylhexyl)sulfosuccinate | 0.01 $g/m^2$ |
| Compound shown below | 0.01 $g/m^2$ |

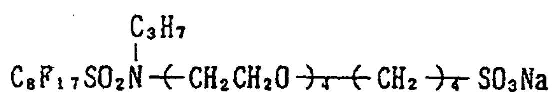

The coercive force of the backing layers thus obtained was 960 Oe.

(4) Heat treatment of the base

After the undercoat layer and the backing layers were applied in the above manner, the base was dried, taken up, and heat-treated at 110 °C for 48 hours.

(5) Preparation of light-sensitive layers

Then, on the side opposite to the backing layers obtained above, was applied light-sensitive layers, described in Hatsumei-Kyokai, Kokai-Giho, Kogi No. 94-6023, page 116, left column, line 17, to page 133, thereby preparing Sample 702 of a multi-layer color light-sensitive material.
The thus-prepared light-sensitive material was cut into strips each having a width of 24 mm and a length of 160 cm. Two perforations of 2 mm square were made at intervals of 5.8 mm, located at the position of 0.7 mm in the

width direction and at one side in the lengthwise direction of the light-sensitive material, respectively. Further, sets of such two perforations were made at intervals of 32 mm. The sample was encased in a plastic film cartridge, as illustrated in Fig. 1 to Fig. 7 of US-A-5 296 887.

The above Sample 702 was processed and evaluated in the same manner as in Example 1 of this application. In passing, after it was exposed to light and processed, the Sample 702 was housed again in the original plastic film cartridge.

In the present invention, the light-sensitive material having a magnetic recording layer on the back surface opposite to the emulsion layer side, gave the similar excellent results as the results of Example 1 of this application.

Example 5

With respect to ethylenediaminetetraacetic acid (EDTA) and Exemplified compounds 1 and 2 according to the present invention, biodegradability of each compound was evaluated, according to 301C Test in the guideline of the OECD Chemicals Test. The results are shown in the Table 3 below.

Table 3

| Decomposition ratio in biodegradability test | | |
|---|---|---|
| Compound | Decomposition ratio | |
| | after 20 days | after 28 days |
| EDTA | 0% | 0% |
| Compound 1 according to the present invention | 80% or more | 80% or more |
| Comparative compound F | 55% | 75% |
| Comparative compound G | 64% | 80% or more |
| Compound 2 according to the present invention | 80% or more | 80% or more |
| Comparative compound H | 47% | 74% |
| Comparative compound I | 57% | 80% or more |

Comparative compound F

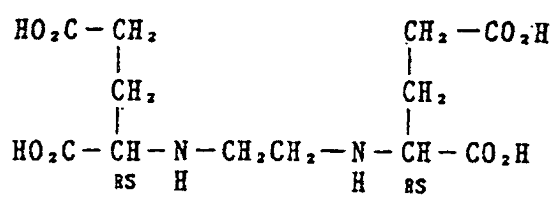

Comparative compound G

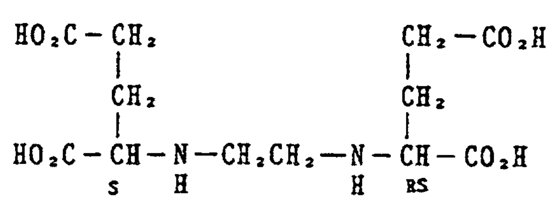

Comparative compound H

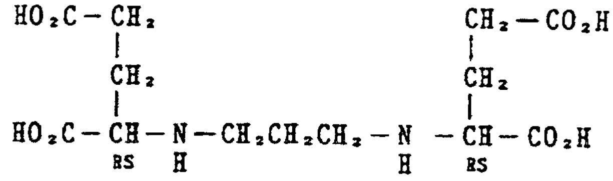

Comparative compound I

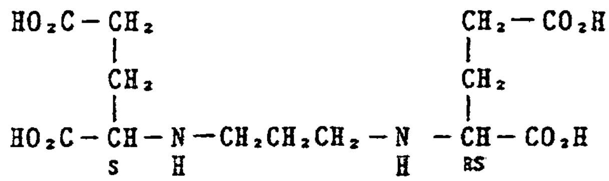

From the results shown in Table 3, when the absolute configuration of the asymmetric carbon is a racemic modification (Comparative compounds F and H), 75% of the decomposition was attained, but it came a little short of 80% which case is generally considered biodegradable. Further, when one absolute configuration is that in an S-form and the other is a racemic modification (Comparative compounds G and I), they were decomposed 80% or more in 28 days, they can be considered as biodegradable compounds. However, in comparison with the SS-form (the compounds according to the present invention), the decomposition rate of these comparative compounds cannot be considered satisfactory. Since the compound used in the present invention is decomposed quickly when released in the environment, the composition according to the present invention is excellent.

Example 6 (Comparative Example)

A paper base both surfaces of which had been coated with a polyethylene resin, was subjected to surface corona discharge treatment; then it was provided with a gelatin undercoat layer containing sodium dodecylbenzensulfonate, and it was successively coated with the first to seventh photographic constitutional layers, to prepare samples of a silver halide color photographic light-sensitive material (000) having the layer configuration shown below. The coating solutions for each photographic constitutional layer were prepared as follows.

(Preparation of Fifth-Layer Coating Solution)

300 g of a cyan coupler (ExC-1), 250 g of a color-image-stabilizer (Cpd-1), 10 g of a color-image-stabilizer (Cpd-9), 10 g of a color-image-stabilizer (Cpd-10), 20 g of a color-image-stabilizer (Cpd-12), 14 g of an ultraviolet absorbing agent (UV-1), 50 g of an ultraviolet absorbing agent (UV-2), 40 g of an ultraviolet absorbing agent (UV-3), and 60 g of an ultraviolet absorbing agent (UV-4) were dissolved in 230 g of a solvent (Solv-6) and 350 ml of ethyl acetate, and the resulting solution was emulsified and dispersed in 6500 g of a 10% aqueous gelatin solution containing 200 ml of 10% sodium dodecylbenzensulfonate, to prepare an emulsified dispersion C.

On the other hand, a silver chlorobromide emulsion C (cubes, a mixture of a large-size emulsion C having an average grain size of 0.50 μm, and a small-size emulsion C having an average grain size of 0.41 pm (1 : 4 in terms of mol of silver), the deviation coefficients of the grain size distributions being 0.09 and 0.11 respectively, and each emulsion having 0.5 mol% of silver bromide locally contained in part of the grain surface whose substrate was made up of silver chloride) was prepared.

To the large-size emulsion C of this emulsion, had been added 6.0 x $10^{-5}$ mol, per mol of silver, of each of red-sensitive sensitizing dyes G, and H shown below, and to the small-size emulsion C of this emulsion, had been added 9.0 x $10^{-5}$ mol, per mol of silver, of each of red-sensitive sensitizing dyes G, and H shown below. The chemical ripening of this emulsion was carried out optimally with a sulfur sensitizer and a gold sensitizer being added.

The above emulsified dispersion C and this silver chlorobromide emulsion C were mixed and dissolved, and

a fifth-layer coating solution was prepared so that it would have the composition shown below. The coating amount of the emulsion is in terms of silver.

The coating solutions for the first layer to forth layer and the sixth layer to seventh layer were prepared in the similar manner as that for the fifth layer coating solution. As the gelatin hardener for each layer, 1-oxy-3,5-dichloro-s-triazine sodium salt was used.

Further, to each layer, were added Ab-1, Ab-2, Ab-3, and Ab-4, so that the total amounts would be 15.0 mg/$m^2$, 60.0 mg/$m^2$, 5.0 mg/$m^2$, and 10.0 mg/$m^2$, respectively.

(Ab-1)
Antiseptics

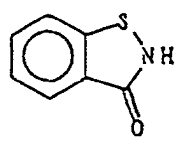

(Ab-2)
Antiseptics

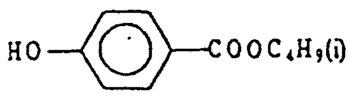

(Ab-3)
Antiseptics

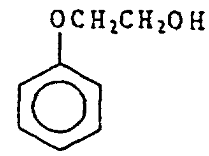

(Ab-4) Antiseptics

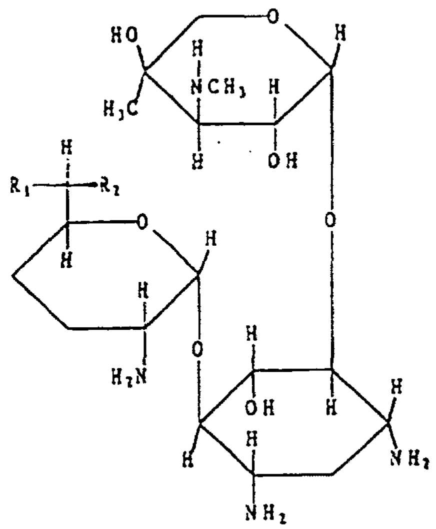

| | $R_1$ | $R_2$ |
|---|---|---|
| a | $-CH_3$ | $-NHCH_3$ |
| b | $-CH_3$ | $-NH_2$ |
| c | $-H$ | $-NH_2$ |
| d | $-H$ | $-NHCH_3$ |

1:1:1:1 mixture of a, b, c, d

For the silver chlorobromide emulsion of each photosensitive emulsion layer, the following spectral sensitizing dyes were used.

(Blue-Sensitive Emulsion Layer)

(Sensitizing dye A)

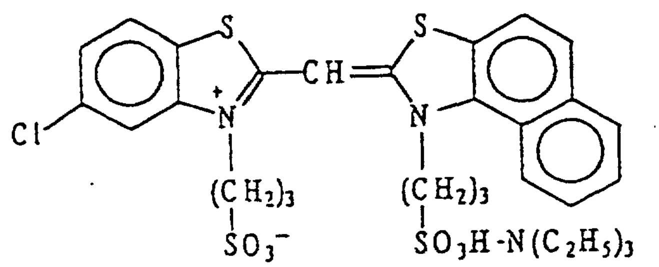

(Sensitizing dye B)

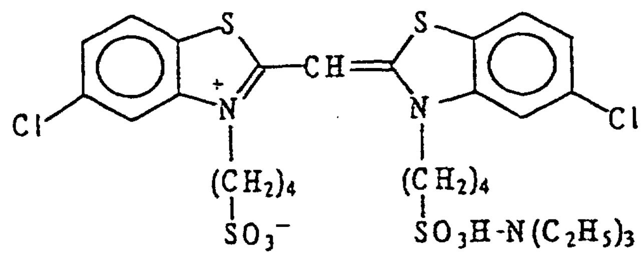

(Sensitizing dye C)

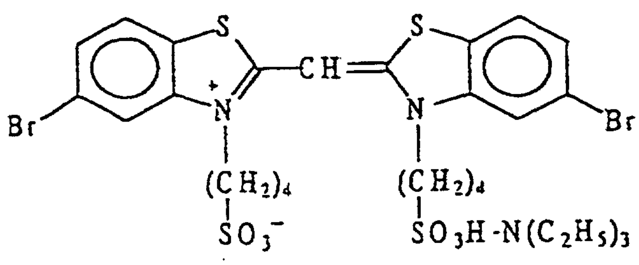

(The sensitizing dyes A, B, and C were added, respectively, to the large-size emulsion, in an amount of 1.4 x $10^{-4}$ mol per mol of the silver halide, and to the small-size emulsion in an amount of 1.7 x $10^{-4}$ per mol of the silver halide.)

(Green-Sensitive Emulsion Layer)

(Sensitizing dye D)

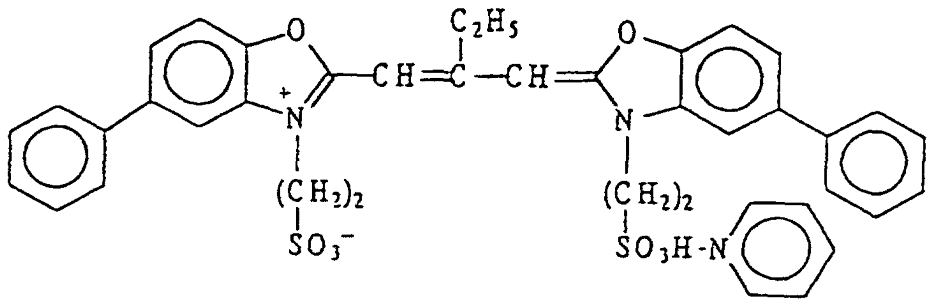

(Sensitizing dye E)

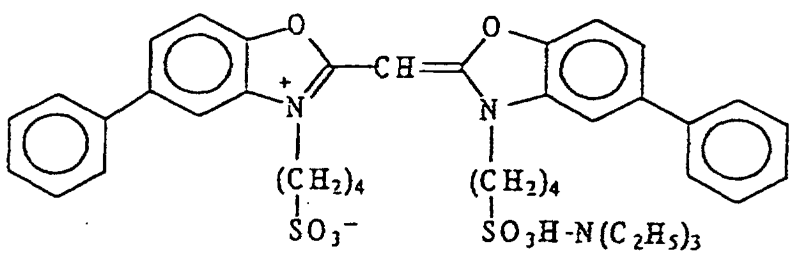

(Sensitizing dye F)

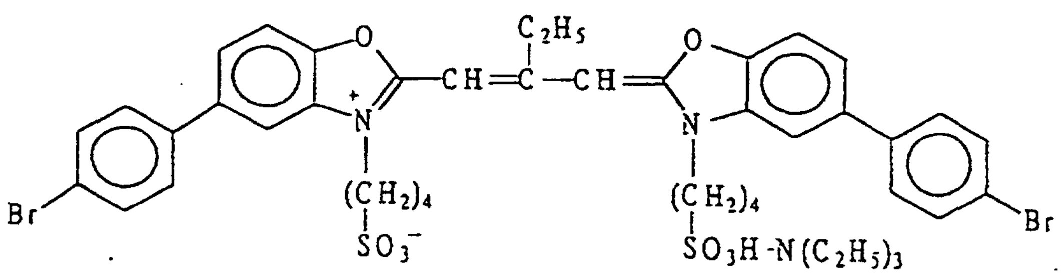

(The sensitizing dye D was added to the large-size emulsion in an amount of 3.0 x $10^{-4}$ mol per mol of the silver halide, and to the small-size emulsion in an amount of 3.6 x $10^{-4}$ mol per mol of the silver halide; the sensitizing dye E was added to the large-size emulsion in an amount of 4.0 x $10^{-5}$ mol per mol of the silver halide, and to the small-size emulsion in an amount of 7.0 x $10^{-5}$ mol per mol of the silver halide; and the sensitizing dye F was added to the large-size emulsion in an amount of 2.0 x $10^{-4}$ mol per mol of the silver halide, and to the small-size emulsion in an

amount of 2.8 x $10^{-4}$ mol per mol of the silver halide.)

(Red-Sensitive Emulsion Layer)

(Sensitizing dye G)

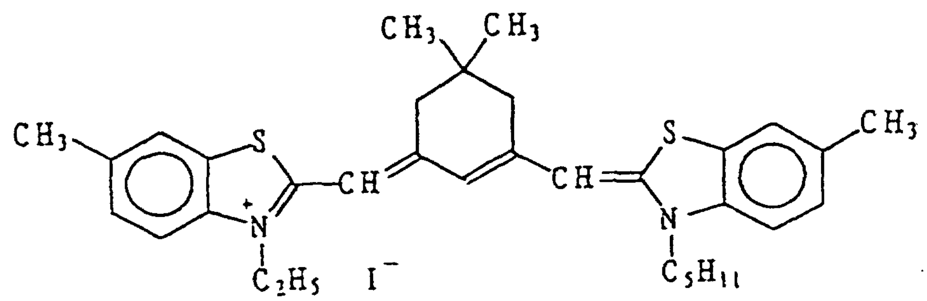

(Sensitizing dye H)

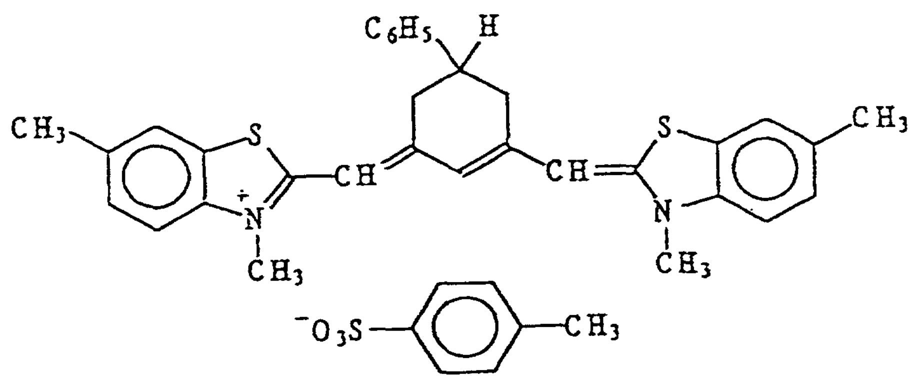

(The sensitizing dyes G and H were added, respectively, to the large-size emulsion, in an amount of 6.0 x $10^{-5}$ mol per mol of the silver halide, and to the small-size emulsion in an amount of 9.0 x $10^{-5}$ per mol of the silver halide.)

Further, the following Compound I was added to the red-sensitive emulsion layer, in an amount of 2.6 x $10^{-3}$ mol per mol of the silver halide.

(Compound I)

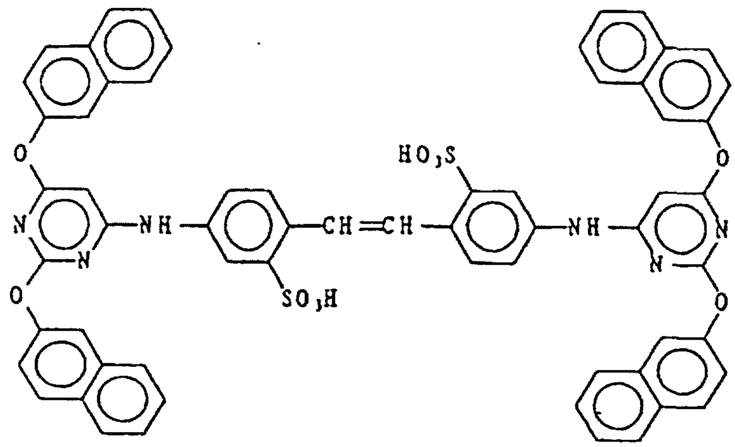

Further, to the blue-sensitive emulsion layer, the green-sensitive emulsion layer, and the red-sensitive emulsion layer, was added 1-(3-methylureidophenyl)-5-mercaptotetrazole in amounts of 3.3 x $10^{-4}$ mol, 1.0 x $10^{-3}$ mol, and 5.9 x $10^{-4}$ mol, per mol of the silver halide, respectively.
Further, to the second layer, the fourth layer, the sixth layer, and the seventh layer, it was added in amounts of 0.2 mg/$m^2$, 0.2 mg/$m^2$, 0.6 mg/$m^2$, and 0.1 mg/$m^2$, respectively.
Further, to the blue-sensitive emulsion layer and the green-sensitive emulsion layer, was added 4-hydroxy-6-methyl-1,3,3a,7-tetrazaindene in amounts of 1 x $10^{-4}$ mol and 2 x $10^{-4}$ mol, respectively, per mol of the silver halide.
To the red-sensitive emulsion layer, was added a copolymer of methacrylic acid and butyl acrylate (1 : 1 in weight ratio; average molecular weight, 200,000 to 400,000) in an amount of 0.05g/$m^2$.
Further, to the second layer, the fourth layer, and the sixth layer, was added disodium catechol-3,5-disulfonate in amounts of 6 mg/$m^2$, 6 mg/$m^2$, and 18 mg/$m^2$, respectively.
Further, to neutralize irradiation, the following dyes were added to the emulsion layers (the coating amount is shown in parentheses).

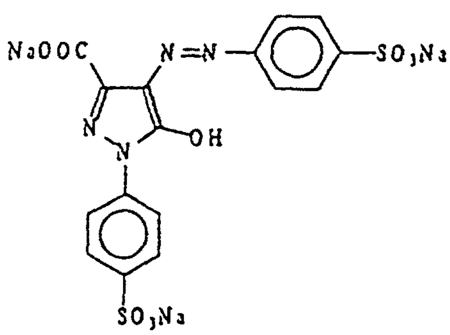

(10mg/m2)

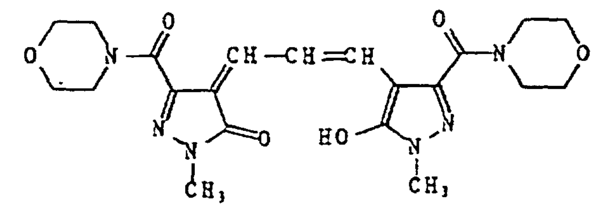

(20mg/m2)

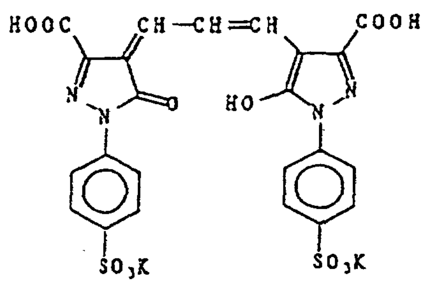

(5mg/m2)

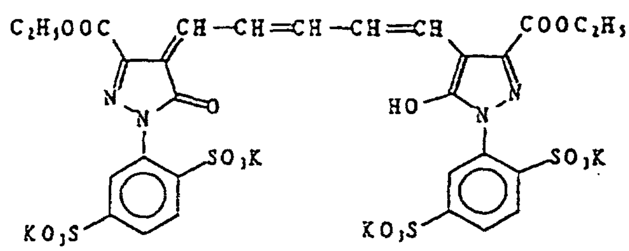

(20mg/m2)

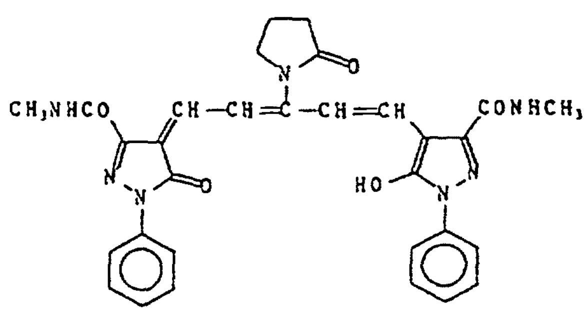

(20mg/m2)

(Layer Constitution)

The composition of each layer is shown below. The numbers show coating amounts ($g/m^2$). In the case of the silver halide emulsion, the coating amount is in terms of silver.

Base

Polyethylene Resin-Laminated Paper

[The polyethylene resin on the first layer side contained a white pigment ($TiO_2$: content of 16 wt%, ZnO:

content of 4 wt%), a fluorescent whitening agent (a mixture of 4,4-bis(benzoxazolyl)stilbene and 4,4'-bis(5-methylbenzoxazolyl)stilbene (8 : 2): content of 0.05 wt%), and a blue dye (ultramarine)]

| First Layer (Blue-Sensitive Emulsion Layer) | |
|---|---|
| A silver chlorobromide emulsion A: cubes, a mixture of a large-size emulsion A having an average grain size of 0.72 µm, and a small-size emulsion A having an average grain size of 0.60 µm (3 : 7 in terms of mol of silver). The deviation coefficients of the grain size distributions were 0.08 and 0.10, respectively, and each emulsion had 0.3 mol % of AgBr locally contained in part of the grain surface whose substrate was made up of silver chloride. | 0.26 |
| Gelatin | 1.35 |
| Yellow coupler (ExY) | 0.62 |
| Color-image stabilizer (Cpd-1) | 0.08 |
| Color-image stabilizer (Cpd-2) | 0.04 |
| Color-image stabilizer (Cpd-3) | 0.08 |
| Solvent (Solv-1) | 0.23 |

| Second Layer (Color-Mixing Inhibiting Layer) | |
|---|---|
| Gelatin | 0.99 |
| Color-mixing inhibitor (Cpd-4) | 0.09 |
| Color-image stabilizer (Cpd-5) | 0.018 |
| Color-image stabilizer (Cpd-6) | 0.13 |
| Color-image stabilizer (Cpd-7) | 0.01 |
| Solvent (Solv-1) | 0.06 |
| Solvent (Solv-2) | 0.22 |

| Third Layer (Green-Sensitive Emulsion Layer) | |
|---|---|
| A silver chlorobromide emulsion B: cubes, a mixture of a large-size emulsion B having an average grain size of 0.45 µm, and a small-size emulsion B having an average grain size of 0.35 µm (1 : 3 in terms of mol of silver). The deviation coefficients of the grain size distributions were 0.10 and 0.08, respectively, and each emulsion had 0.4 mol % of AgBr locally contained in part of the grain surface whose substrate was made up of silver chloride. | 0.14 |
| Gelatin | 1.36 |
| Magenta coupler (ExM) | 0.15 |
| Ultraviolet absorbing agent (UV-1) | 0.05 |
| Ultraviolet absorbing agent (UV-2) | 0.03 |
| Ultraviolet absorbing agent (UV-3) | 0.02 |
| Ultraviolet absorbing agent (UV-4) | 0.04 |
| Color-image stabilizer (Cpd-2) | 0.02 |
| Color-image stabilizer (Cpd-4) | 0.002 |
| Color-image stabilizer (Cpd-6) | 0.09 |
| Color-image stabilizer (Cpd-8) | 0.02 |
| Color-image stabilizer (Cpd-9) | 0.03 |
| Color-image stabilizer (Cpd-10) | 0.01 |
| Color-image stabilizer (Cpd-11) | 0.0001 |
| Solvent (Solv-3) | 0.11 |
| Solvent (Solv-4) | 0.22 |
| Solvent (Solv-5) | 0.20 |

| Fourth Layer (Color-Mixing Inhibiting Layer) | |
|---|---|
| Gelatin | 0.71 |
| Color-mixing inhibitor (Cpd-4) | 0.06 |
| Color-image stabilizer (Cpd-5) | 0.013 |
| Color-image stabilizer (Cpd-6) | 0.10 |
| Color-image stabilizer (Cpd-7) | 0.007 |
| Solvent (Solv-1) | 0.04 |
| Solvent (Solv-2) | 0.16 |

| Fifth Layer (Red-Sensitive Emulsion Layer) | |
|---|---|
| A silver chlorobromide emulsion C: cubes, a mixture of a large-size emulsion C having an average grain size of 0.50 µm, and a small-size emulsion C having an average grain size of 0.41 µm (1 : 4 in terms of mol of silver). The deviation coefficients of the grain size distributions were 0.09 and 0.11, respectively, and each emulsion had 0.5 mol % of silver bromide locally contained in part of the grain surface whose substrate was made up of silver chloride. | 0.20 |
| Gelatin | 1.11 |
| Cyan coupler (ExC-1) | 0.30 |
| Ultraviolet absorbing agent (UV-1) | 0.14 |
| Ultraviolet absorbing agent (UV-2) | 0.05 |
| Ultraviolet absorbing agent (UV-3) | 0.04 |
| Ultraviolet absorbing agent (UV-4) | 0.06 |
| Color-image stabilizer (Cpd-1) | 0.25 |
| Color-image stabilizer (Cpd-9) | 0.01 |
| Color-image stabilizer (Cpd-10) | 0.01 |
| Color-image stabilizer (Cpd-12) | 0.02 |
| Solvent (Solv-6) | 0.23 |

| Sixth Layer (Ultraviolet Absorbing Layer) | |
|---|---|
| Gelatin | 0.66 |
| Ultraviolet absorbing agent (UV-1) | 0.19 |
| Ultraviolet absorbing agent (UV-2) | 0.06 |
| Ultraviolet absorbing agent (UV-3) | 0.06 |
| Ultraviolet absorbing agent (UV-4) | 0.05 |
| Ultraviolet absorbing agent (UV-5) | 0.09 |
| Solvent (Solv-7) | 0.25 |

| Seventh Layer (Protective Layer) | |
|---|---|
| Gelatin | 1.00 |
| Acryl-modified copolymer of polyvinyl alcohol (modification degree: 17 %) | 0.04 |
| Liquid paraffin | 0.02 |
| Surface-active agent (Cpd-13) | 0.01 |

(ExY) Yellow coupler

60:40 mixture of

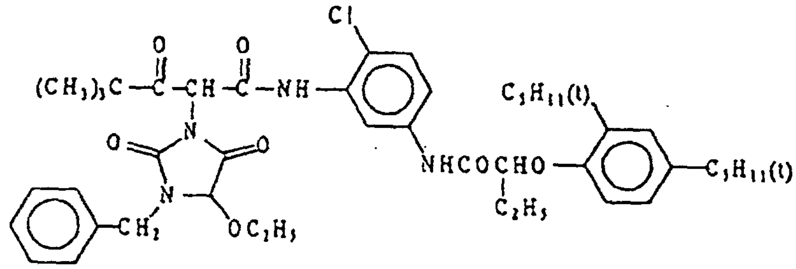

and

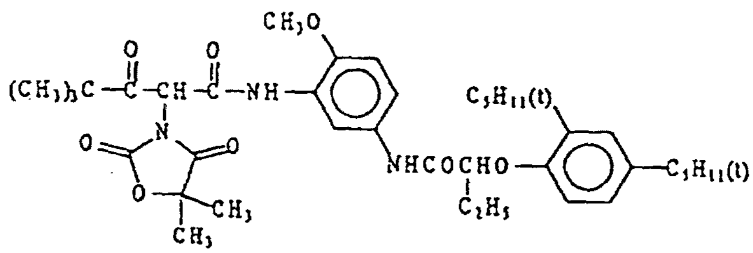

(ExM) Magenta coupler

60:40 mixture of

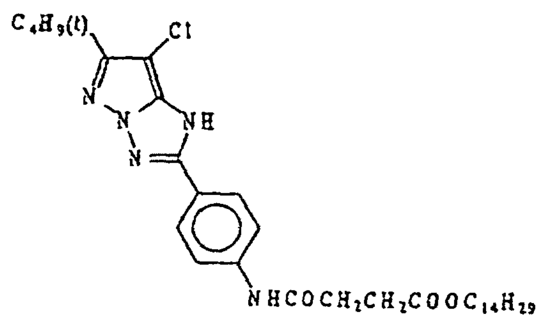

and

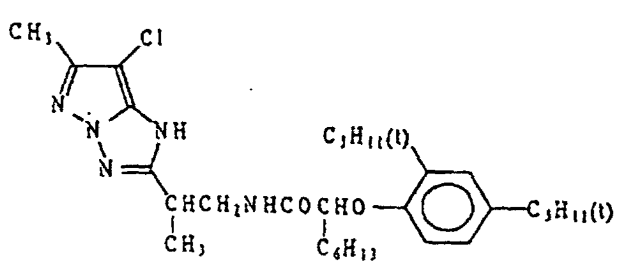

(ExC-1) Cyan coupler

15:85 mixture of

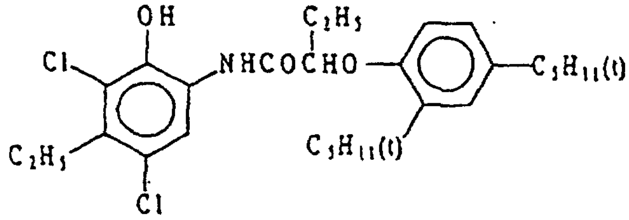

and

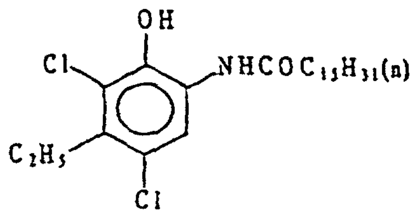

(ExC-2) Cyan coupler

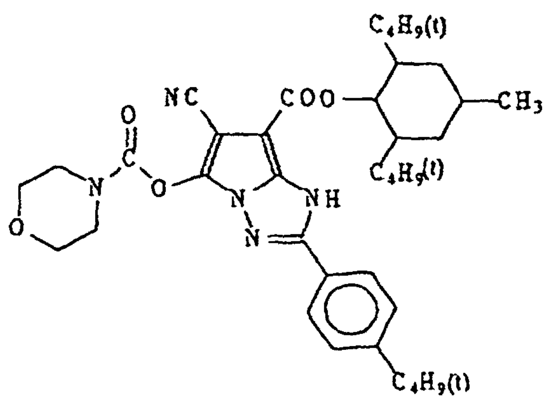

(ExC-3) Cyan coupler

50:25:25 mixture of

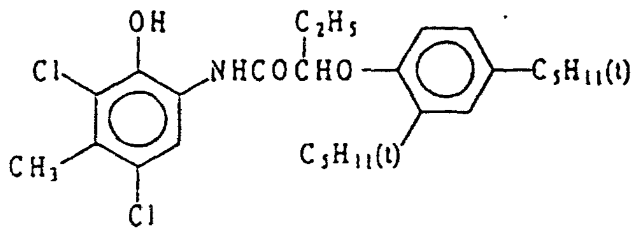

and

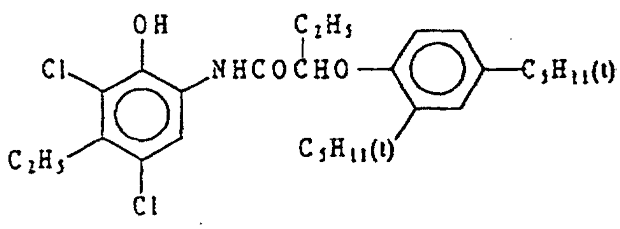

and

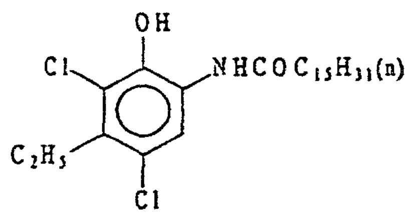

(Cpd-1) Color-image stabilizer

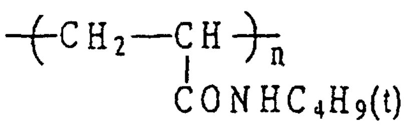

number average molecular weight 60,000

(Cpd−2) Color-image stabilizer

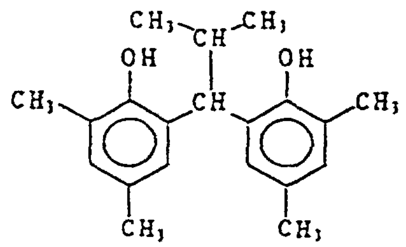

(Cpd−3) Color-image stabilizer

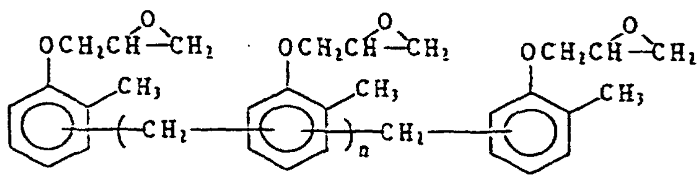

n = 7 ∼ 8 (average)

(Cpd−4) Color-mixing inhibitor
1:1:1 mixture of

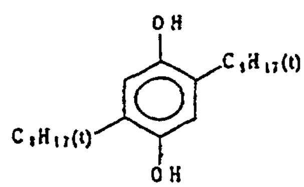

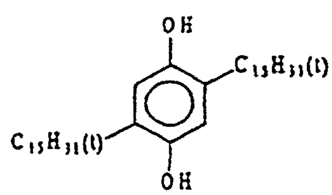

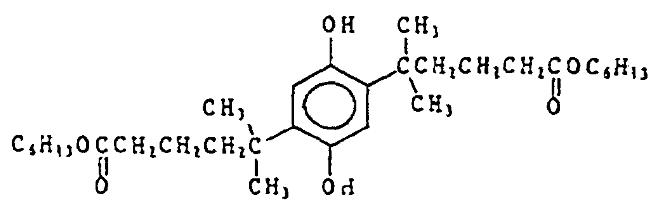

(Cpd−5) Color-mixing inhibiting auxiliary

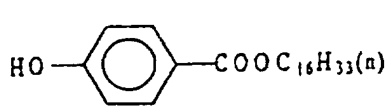

(Cpd-6) Stabilizer

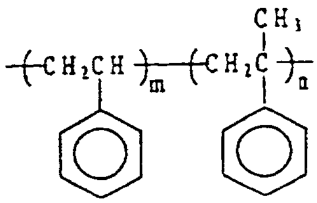

number average molecular weight 600

m/n=10/90

(Cpd-7) Color-mixing inhibitor

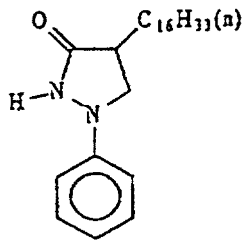

(Cpd-8) Color-image stabilizer

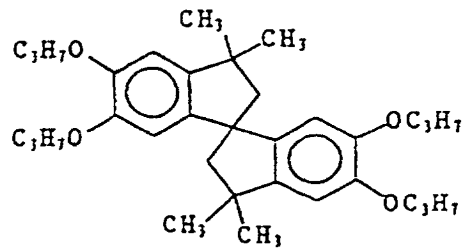

(Cpd-9) Color-image stabilizer

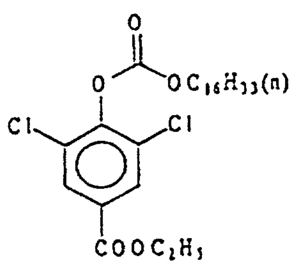

(Cpd-10) Color-image stabilizer

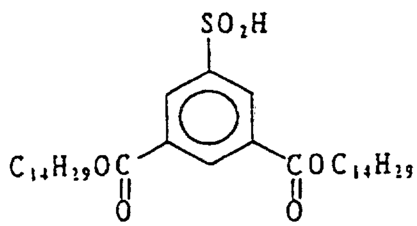

(Cpd-11)

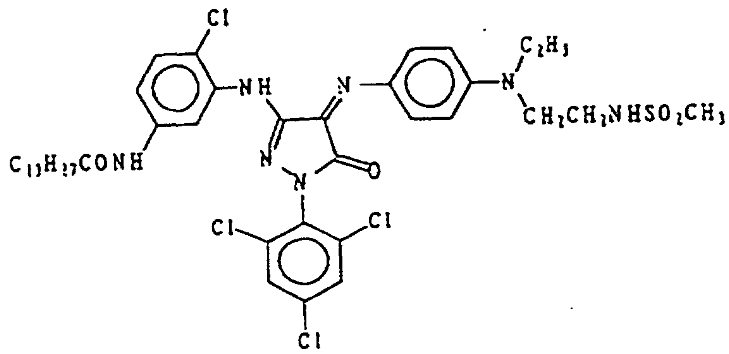

(Cpd-12) Color-image stabilizer

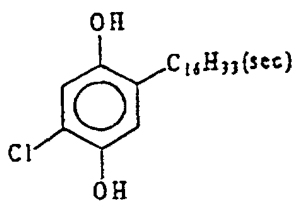

(Cpd-13) Surface-active agent

7:3 mixture of

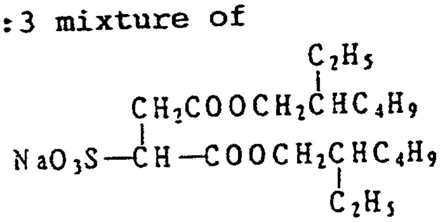

and

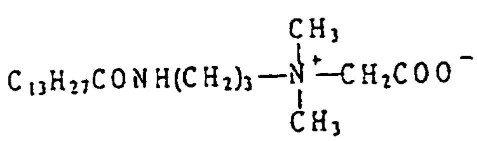

(Cpd-14)

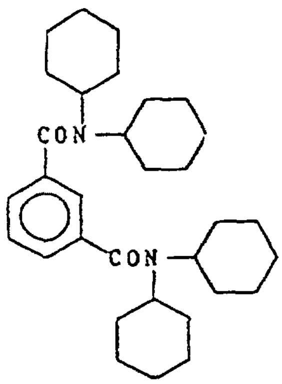

(Cpd-15)
1:1 mixture of

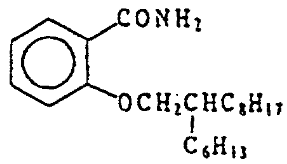

and

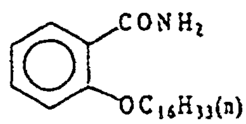

(Cpd-16)

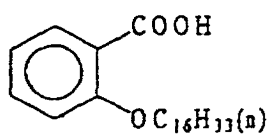

(Cpd-17)

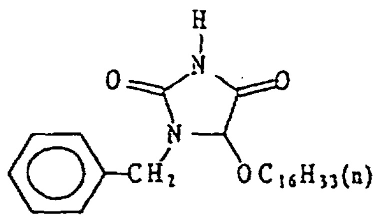

(Cpd-18)

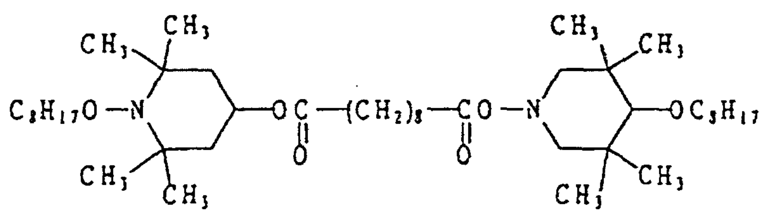

(UV−1) UV absorbing agent

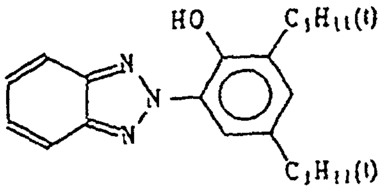

(UV−2) UV absorbing agent

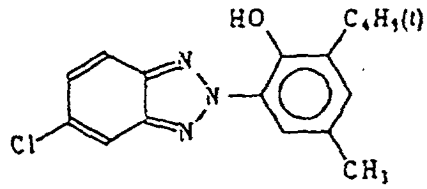

(UV−3) UV absorbing agent

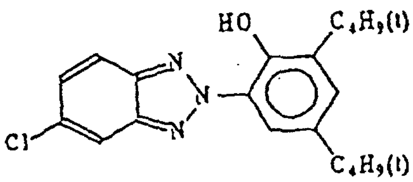

(UV−4) UV absorbing agent

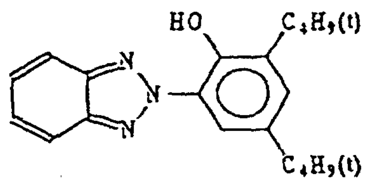

(UV−5) UV absorbing agent

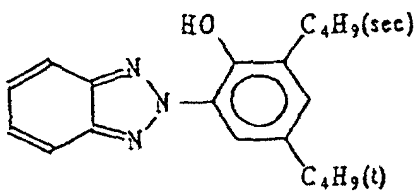

(Solv−1)

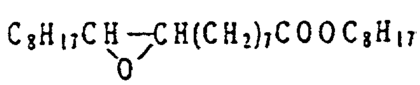

(Solv−2)

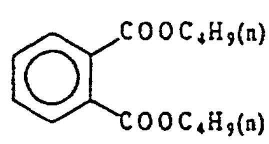

(Solv-3)

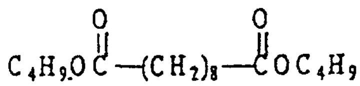

(Solv-4)

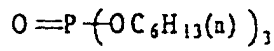

(Solv-5)

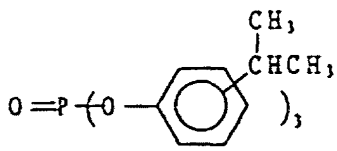

(Solv-6)

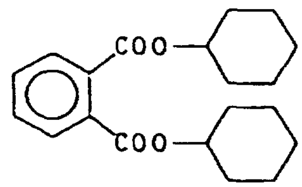

(Solv-7)

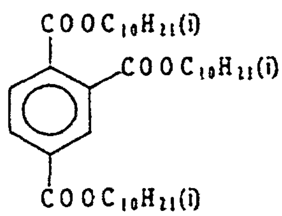

(Solv-8)

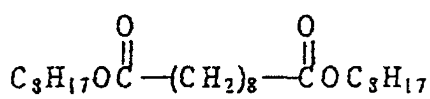

(Solv-9)

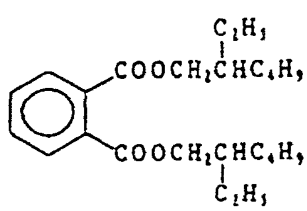

The processing solutions having the below shown composition were prepared.

| (Color-developer) | Tank Solution | Replenisher |
|---|---|---|
| Water | 800.0ml | 800.0 ml |
| Triethanolamine (80%) | 9.0 g | 12.0 g |
| Potassium hydroxide | 4.0 g | 6.0 g |
| Diethylenetriaminepentaacetic acid | 0.5 g | 0.8 g |
| Ethylenediaminetetraacetic acid | 2.0 g | 3.0 g |
| Potassium chloride | 1.5 g | - |

(continued)

| (Color-developer) | Tank Solution | Replenisher |
|---|---|---|
| Sodium sulfite | 0.2 g | 0.2 g |
| Potassium carbonate | 18.0 g | 25.5 g |
| Sodium bicarbonate | 4.1 g | 8.0 g |
| N-Ethyl-N-(β-methanesulfonamidoethyl)-3-methyl-4-aminoaniline sulfate | 5.0 g | 7.5 g |
| Water to make | 1000 ml | 1000 ml |
| pH (25°C) | 10.05 | 10.45 |

| (Bleach-Fix solution) | Tank solution | Replenisher |
|---|---|---|
| Ammonium thiosulfate (70 w/v%) | 120 ml | 144 ml |
| Sodium sulfite | 10.0 g | 12.0 g |
| Water | 300 ml | 300 ml |
| Chelating agent A described in Table 4 | 0.060 mol | 0.072 mol |
| Chelating agent B described in Table 4 | 0.050 mol | 0.060 mol |
| Ferric nitrate nonahydrate | 40.4 g | 48.4 g |
| 90% Acetic acid | 7.0 g | 8.4 g |
| 57% Glycolic acid | 13.0 g | 15.6 g |
| Imidazole | 14.0 g | 16.8 g |
| Water to make | 1000 ml | 1000 ml |
| pH (25 °C) | 6.8 | 6.0 |
| (pH was adjusted by potassium hydroxide and nitric acid) | | |

(Washing water) (Both mother solution and replenisher) Tap water for domestic use

In the continuous processing of each of the samples using the color-developer, the overflow from the bleach-fix solution is recovered. When the recovered amount reached to be 2 times the tank volume (20 liter), the overflow solution was regenerated by adding the following bleach-fixing-solution-regenerating agent, to be re-used as a replenisher of the bleach-fix solution.

The recipe of the bleach-fixing-solution-regenerating agent is shown below.

| (Bleach-fix-solution-regenerating agent) (Amount added per liter of the recovered overflow solution for regeneration) | |
|---|---|
| Ammonium thiosulfate (70 w/v%) | 24 ml |
| Sodium bisulfite | 12.0 g |
| Chelating agent A described in Table 4 | 0.018 mol |
| Chelating agent B described in Table 4 | 0.015 mol |
| Ferric nitrate nonahydrate | 0.030 mol |
| Imidazole | 4.2 g |
| 57% Glycolic acid | 4.0 g |
| Glacial acetic acid | 2.8 g |
| pH | pH 5.5 |
| (pH was adjusted by potassium hydrate) | |

| Processing step ($P_{Blix}$) | | | | |
|---|---|---|---|---|
| Processing step | Processing | | Replenishing rate* | Tank Volume |
| | temperature | time | | |
| Color developing | 35 °C | 45 sec | 161 ml | 10 l |
| Bleach-Fixing | 33 to 37 °C | 45 sec | 134 ml | 10 l |
| Washing (1) | 24 to 34 °C | 20 sec | - | 5 l |
| Washing (2) | 24 to 34 °C | 20 sec | - | 5 l |
| Washing (3) | 24 to 34 °C | 20 sec | 350 ml | 5 l |
| Drying | 60 to 90 °C | 60 sec | | |

* The replenishment rate was the amount per $m^2$ of the light-sensitive material.
(The washing was conducted in a 3-tank counter-current system of Washing (3) → (1))

To investigate the amount of residual silver after the processing, Sample 000 was exposed uniformly to light, so that the gray density would be 2.2, and then it was processed in the above processing step ($P_{Blix}$). The amount of residual silver was measured by fluorescent X-ray analysis.

To examine the Blix fading, Sample 000, which was subjected to gradation exposure through a wedge and then was processed in the processing step $P_{Blix}$, was processed again in the below reprocessing step $P_{EDTA}$. By comparing the Dmax values measured with red light (R light) before and after the reprocessing, the extent of the Blix fading was examined.

| Reprocessing step ($P_{EDTA}$) | | | | |
|---|---|---|---|---|
| Processing step | Processing | | Replenishing rate* | Tank Volume |
| | temperature | time | | |
| Bleach-Fixing | 33 to 37 °C | 45 sec | 134 ml** | 10 liter |
| Washing (4) | 24 to 34 °C | 20 sec | - | 5 liter |
| Washing (5) | 24 to 34 °C | 20 sec | - | 5 liter |
| Washing (6) | 24 to 34 °C | 20 sec | 350 ml | 5 liter |
| Drying | 60 to 90 °C | 60 sec | | |

* The replenishment rate was the amount per $m^2$ of the light-sensitive material.
(The washing was conducted in a 3-tank counter-current system of Washing (6) → (4))

The process ($P_{Blix}$) was started by using the above processing solutions and placing the tank solutions in respective processing tanks in the above processing step $P_{Blix}$, and it was continued by adding the replenishers to the respective tanks in accordance with the processed amount. The process was carried out until the accumulated replenishment rate reached three times the tank volume. The results of the processing carried out at this point are shown in Table 4. The reprocess ($P_{EDTA}$) was carried out in the above reprocessing step $P_{EDTA}$ with the chelating agents A and B of the bleach-fix solution changed to ethylenediaminetetraacetic acid. Other components were the same as those in $P_{Blix}$, provided that the reprocess ($P_{EDTA}$) was carried out with the respective solutions being fresh.

Further, to evaluate the effect of the addition of imidazole, by removing only this component from the bleach-fix solution and the bleach-fix-solution-regenerating agent, a bleach-fix solution and a bleach-fix-solution-regenerating agent were prepared and the evaluation was carried out. Further, to evaluate the effect of the combined use of the compound according to the present invention and ethylenediaminetetraacetic acid iron salt, Chelating agent B in the bleach-fix solution and the bleach-fix-solution-regenerating agent was replaced with ethylenediaminetetraacetic acid and the evaluation was carried out.

As for the evaluation of the precipitation in the washing bath, after the processing, 100 ml of the aqueous solution of the washing bath (1) was allowed to stand for one month in the dark at room temperature (25 °C), and the formation of precipitation (brown) was visually evaluated.

The results are shown in Table 4.

Table 4

| No. | Chelating agent A | Chelating agent B | Addition of imidazole | Residual silver amount $\mu g/cm^2$ | Blix fading ΔDmax(R) | Precipitation* | Remarks |
|---|---|---|---|---|---|---|---|
| 401 | Comparative compound A | Comparative compound A | done | 2.8 | 0.00 | ○ | Comparative example |
| 402 | Comparative compound B | Comparative compound B | done | 11.5 | 0.00 | × | Comparative example |
| 403 | Comparative compound C | Comparative compound C | done | 2.3 | 0.21 | ×× | Comparative example |
| 404 | Comparative compound D | Comparative compound D | done | 2.0 | 0.17 | ××× | Comparative example |
| 405 | Comparative compound E | Comparative compound E | done | 2.7 | 0.17 | × | Comparative example |
| 406 | Exemplified compound 1 | Exemplified compound 1 | done | 1.0 | 0.00 | ○ | Comparative Example |
| 407 | Exemplified compound 1 | Exemplified compound 1 | none | 1.2 | 0.00 | × | Comparative Example |
| 408 | Exemplified compound 1 | ethylenediamine-tetraacetic acid | done | 0.5 | 0.00 | ○ | Comparative Example |
| 409 | Exemplified compound 1 | ethylenediamine-tetraacetic acid | none | 0.6 | 0.00 | ○ | Comparative Example |

Note: *"○" designates no precipitation; "×" designates occurrence of precipitation, and the more the number of "×" is, the more the amount of precipitation formed is.

< Comparative Example >

Comparative compounds A, B, C, D, and E were the same as those in Example 1.

Having described our invention as related to the present embodiments, it is our intention that the invention

not be limited by any of the details of the description, unless otherwise specified, but rather be construed broadly within its scope as set out in the accompanying claims.

## Claims

**1.** A photographic bleach-fix solution, comprising m-carboxybenzene sulfinic acid or p-aminobenzene sulfinic acid and a Fe(III) complex salt of a compound represented by the following formula (I):

formula (I)

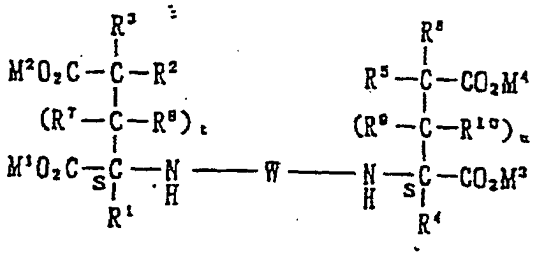

wherein $R^1$, $R^2$, $R^3$, $R^4$, $R^5$, $R^6$, $R^7$, $R^8$, $R^9$, and $R^{10}$ each represent a hydrogen atom, an aliphatic group, an aromatic group, or a hydroxyl group; W represents a divalent linking group containing a carbon atom; $M^1$, $M^2$, $M^3$, and $M^4$ each represent a hydrogen atom or a cation; t and u each represent an integer of 1 to 6; and S indicates that the absolute configuration of an asymmetric carbon is the S-configuration,

**2.** The photographic bleach-fix solution according to claim 1, further comprising a Fe(III) complex salt of ethylenediamine tetraacetic acid.

**3.** The photographic bleach-fix solution according to claim 2, further comprising imidazole.

**4.** A method of processing a silver halide color photographic light-sensitive material, comprising processing a silver halide color photographic light-sensitive material exposed to light imagewise with a bleach-fix solution according to any one of claims 1 to 3.

**5.** The method of processing as claimed in claim 4 wherein the bleaching agent comprises ethylenediaminetetraacetic acid Fe(III) complex salt in an amount of ½ or less in the mole fraction to all the bleaching agent.

## Patentansprüche

**1.** Fotografische Bleichfixierlösung, die m-Carboxybenzolsulfinsäure oder p-Aminobenzolsulfinsäure und ein Fe(III) -Komplexsalz einer Verbindung der folgenden Formel (I) umfasst:

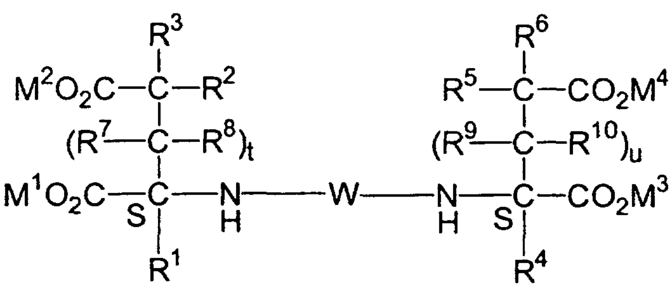

worin $R^1$, $R^2$, $R^3$, $R^4$, $R^5$, $R^6$, $R^7$, $R^8$, $R^9$ und $R^{10}$ jeweils ein Wasserstoffatom, eine aliphatische Gruppe, eine aromatische Gruppe oder eine Hydroxylgruppe repräsentieren, W ist eine divalente Verbindungsgruppe, die ein

Kohlenstoffatom enthält, $M^1$, $M^2$, $M^3$ und $M^4$ repräsentieren jeweils ein Wasserstoffatom oder ein Kation; t und u sind jeweils eine ganze Zahl von 1-6 und S gibt an, dass die absolute Konfiguration des asymmetrischen Kohlenstoffs die S-Konfiguration ist.

**2.** Fotografische Bleichfixierlösung gemäss Anspruch 1, die ferner ein Fe(III)-Komplexsalz von Ethylendiamintetraessigsäure umfasst.

**3.** Fotografische Bleichfixierlösung gemäss Anspruch 2, die ferner Imidazol umfasst.

**4.** Verfahren zur Verarbeitung eines lichtempfindlichen farbfotografischen Silberhalogenidmaterials, das die Verarbeitung eines bildweise mit Licht belichteten, lichtempfindlichen, farbfotografischen Silberhalogenidmaterials mit einer Bleichfixierlösung gemäss mindestens einem der Ansprüche 1 bis 3 umfasst.

**5.** Verfahren zur Verarbeitung gemäss Anspruch 4, worin das Bleichmittel Ethylendiamintetraessigsäure-Fe(III)-Komplexsalz in einer Menge von der Hälfte oder weniger, bezogen auf den Molanteil aller Bleichmittel, umfasst.

## Revendications

**1.** Solution de blanchiment-fixage photographique, comprenant de l'acide m-carboxybenzènesulfinique ou de l'acide p-aminobenzènesulfinique et un sel de complexe de Fe(III) d'un composé représenté par la formule suivante (I) :

Formule (I)

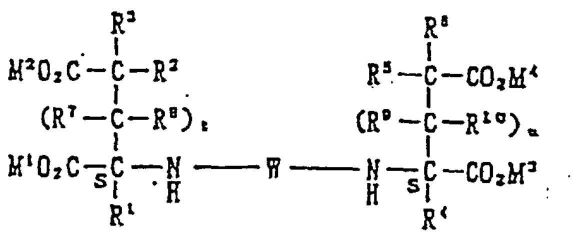

dans laquelle $R^1$, $R^2$, $R^3$, $R^4$, $R^5$, $R^6$, $R^7$, $R^8$, $R^9$ et $R^{10}$ représentent chacun un atome d'hydrogène, un groupe aliphatique, un groupe aromatique, ou un groupe hydroxyle ; W représente un groupe de liaison divalent contenant un atome de carbone ; $M^1$, $M^2$, $M^3$, et $M^4$ représentent chacun un atome d'hydrogène ou un cation ; t et u représentent chacun un nombre entier de 1 à 6 ; et S indique que la configuration absolue d'un carbone asymétrique est la configuration S.

**2.** Solution de blanchiment-fixage photographique selon la revendication 1, comprenant en plus un sel de complexe de Fe(III) d'acide éthylènediaminetétraacétique.

**3.** Solution de blanchiment-fixage photographique selon la revendication 2, comprenant en plus un imidazole.

**4.** Procédé de traitement d'un matériau photographique couleur sensible à la lumière à l'halogénure d'argent, comprenant le traitement d'un matériau photographique couleur sensible à la lumière à l'halogénure d'argent exposé à la lumière correspondant à une image avec une solution de blanchiment-fixage selon l'une quelconque des revendications 1 à 3.

**5.** Procédé de traitement comme revendiqué dans la revendication 4 dans lequel l'agent de blanchiment comprend un sel de complexe de Fe(III) d'acide éthylènediaminetétraacétique en une quantité de ½ ou moins dans la fraction molaire par rapport à la totalité de l'agent de blanchiment.